(19) Europäisches Patentamt / European Patent Office / Office européen des brevets

(11) EP 2 908 642 B1

# (12) EUROPEAN PATENT SPECIFICATION

(45) Date of publication and mention of the grant of the patent:
**23.02.2022 Bulletin 2022/08**

(21) Application number: **13777292.7**

(22) Date of filing: **17.10.2013**

(51) International Patent Classification (IPC):
***A01P 21/00*** $^{(2006.01)}$ ***A01N 43/56*** $^{(2006.01)}$

(52) Cooperative Patent Classification (CPC):
**A01N 43/56; A01P 21/00; C07D 231/10**

(86) International application number:
**PCT/EP2013/071733**

(87) International publication number:
**WO 2014/060519 (24.04.2014 Gazette 2014/17)**

## (54) METHOD FOR ENHANCING TOLERANCE TO ABIOTIC STRESS IN PLANTS BY USING CARBOXAMIDE OR THIOCARBOXAMIDE DERIVATIVES

VERFAHREN ZUR VERBESSERUNG DER TOLERANZ GEGENÜBER ABIOTISCHEM STRESS BEI PFLANZEN MITTELS CARBOXAMID- ODER THIOCARBOXAMIDDERIVATEN

PROCÉDÉ D'AMÉLIORATION DE LA TOLÉRANCE DES PLANTES AUX STRESS ABIOTIQUES À L'AIDE DE DÉRIVÉS CARBOXAMIDE OU THIOCARBOXAMIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2012 EP 12356022**
**27.11.2012 US 201261730282 P**

(43) Date of publication of application:
**26.08.2015 Bulletin 2015/35**

(73) Proprietor: **Bayer CropScience AG**
**40789 Monheim (DE)**

(72) Inventors:
- **CRISTAU, Pierre**
  **F-69009 Lyon (FR)**
- **DITTGEN, Jan**
  **60316 Frankfurt (DE)**
- **SCHMUTZLER, Dirk**
  **65795 Hatternsheim (DE)**

(74) Representative: **BIP Patents**
**c/o Bayer Intellectual Property GmbH**
**Alfred-Nobel-Straße 10**
**40789 Monheim am Rhein (DE)**

(56) References cited:
**EP-A1- 2 251 331**
**WO-A2-2010/130767**
**US-A1- 2011 196 000**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to the use of N-cyclopropyl-N-[substituted-benzyl]-3-(difluoromethyl)-5-fluoro-1-methyl-1H-pyrazole-4-carboxamide or thiocarboxamide derivatives and/or salts thereof for enhancing stress tolerance to abiotic stress in plants and to the associated enhancement in plant growth and/or increase in plant yield, and to associated methods

N-cyclopropyl-N-[substituted-benzyl]-3-(difluoromethyl)-5-fluoro-1-methyl-1H-pyrazole-4-carboxamide or thiocarboxamide derivatives, their preparation from commercially available materials and their use as fungicides are disclosed in WO2007/087906, WO2009/016220, WO2010/130767 and EP2251331. It is also known that these compounds can be used as fungicides and mixed with other fungicides or insecticides (cf. patent applications PCT/EP2012/001676 and PCT/EP2012/001674).

It is known that plants react to natural stress conditions, for example heat and aridity or lack of water (though aridity and lack of water similarly cause drought stress), cold, salinity, UV light, injury, pathogenic attack (viruses, bacteria, fungi, insects), etc., but also to herbicides, with specific or unspecific defense mechanisms [Pflanzenbiochemie (Plant Biochemistry), p. 393-462 , Spektrum Akademischer Verlag, Heidelberg, Berlin, Oxford, Hans W. Heldt, 1996.; Biochemistry and Molecular Biology of Plants, p. 1102-1203, American Society of Plant Physiologists, Rockville, Maryland, eds. Buchanan, Gruissem, Jones, 2000].

In plants, there is knowledge of numerous proteins, and the genes which code for them, which are involved in defense reactions to abiotic stress (for example cold, heat, drought, salt, flooding). Some of these form part of signal transduction chains (for example transcription factors, kinases, phosphatases) or cause a physiological response of the plant cell (for example ion transport, deactivation of reactive oxygen species). The signaling chain genes of the abiotic stress reaction include transcription factors of the DREB and CBF classes (Jaglo-Ottosen et al., 1998, Science 280: 104-106). The reaction to salinity stress involves phosphatases of the ATPK and MP2C types. In addition, in the event of salinity stress, the biosynthesis of osmolytes such as proline or sucrose is often activated. This involves, for example, sucrose synthase and proline transporter (Hasegawa et al., 2000, Annu Rev Plant Physiol Plant Mol Biol 51: 463-499). The stress defense of the plants to cold and drought uses some of the same molecular mechanisms. There is a known accumulation of what are called late embryogenesis abundant proteins (LEA proteins), which include the dehydrins as an important class (Ingram and Bartels, 1996, Annu Rev Plant Physiol Plant Mol Biol 47: 277-403, Close, 1997, Physiol Plant 100: 291-296). These are chaperones which stabilize vesicles, proteins and membrane structures in stressed plants (Bray, 1993, Plant Physiol 103: 1035-1040). In addition, there is frequently induction of aldehyde dehydrogenases, which deactivate the reactive oxygen species (ROS) which form in the event of oxidative stress (Kirch et al., 2005, Plant Mol Biol 57: 315-332). Heat shock factors (HSF) and heat shock proteins (HSP) are activated in the event of heat stress and play a similar role here as chaperones to that of dehydrins in the event of cold and drought stress (Yu et al., 2005, Mol Cells 19: 328-333).

A number of plant-endogeneous signaling substances involved in stress tolerance or pathogen defense are already known. Examples here include salicylic acid, benzoic acid, jasmonic acid or ethylene [Biochemistry and Molecular Biology of Plants, p. 850-929, American Society of Plant Physiologists, Rockville, Maryland, eds. Buchanan, Gruissem, Jones, 2000]. Some of these substances or the stable synthetic derivatives and derived structures thereof are also effective on external application to plants or in seed dressing, and activate defense reactions which cause elevated stress tolerance or pathogen tolerance of the plant [Sembdner, and Parthier, 1993, Ann. Rev. Plant Physiol. Plant Mol. Biol. 44: 569-589].

It is additionally known that chemical substances can increase the tolerance of plants to abiotic stress. Such substances are applied by seed dressing, by leaf spraying or by soil treatment. For instance, an increase in abiotic stress tolerance of crop plants by treatment with elicitors of systemic acquired resistance (SAR) or abscisic acid derivatives is described (Schading and Wei, WO-200028055, Abrams and Gusta, US-5201931, Churchill et al., 1998, Plant Growth Regul 25: 35-45) or azibenzolar-S-methyl. Similar effects are also observed on application of fungicides, especially from the group of the strobilurins or of the succinate dehydrogenase inhibitors, and are frequently also accompanied by an increase in yield (Draber et al., DE-3534948, Bartlett et al., 2002, Pest Manag Sci 60: 309). It is likewise known that the herbicide glyphosate in low dosage stimulates the growth of some plant species (Cedergreen, Env. Pollution 2008, 156, 1099).

In addition, effects of growth regulators on the stress tolerance of crop plants have been described (Morrison and Andrews, 1992, J Plant Growth Regul 11: 113-117, RD-259027). In the event of osmotic stress, a protective effect has been observed as a result of application of osmolytes, for example glycine betaine or the biochemical precursors thereof, e.g. choline derivatives (Chen et al., 2000, Plant Cell Environ 23: 609-618, Bergmann et al., DE-4103253). The effect of antioxidants, for example naphthols and xanthines, to increase abiotic stress tolerance in plants has also already been described (Bergmann et al., DD-277832, Bergmann et al., DD-277835). However, the molecular causes of the antistress action of these substances are substantially unknown.

It is additionally known that the tolerance of plants to abiotic stress can be increased by a modification of the

activity of endogenous poly-ADP-ribose polymerases (PARP) or poly-(ADP-ribose) glycohydrolases (PARG) (de Block et al., The Plant Journal, 2004, 41, 95; Levine et al., FEBS Lett. 1998, 440, 1; WO00/04173; WO04/090140).

US 2011/0196000 A1 disloses the use of succinate dehydrogenase inhibitors for increasing the biomass of a plant and simultaneously increasing its tolerance towards abiotic stress factors. However, said document does not disclose the use of the compounds of the present invention.

It is thus known that plants possess several endogenous reaction mechanisms which can bring about effective defense against a wide variety of different harmful organisms and/or natural abiotic stress.

Since the ecologic and economic demands on modern crop treatment compositions are increasing constantly, for example with respect to toxicity, selectivity, application rate, formation of residues and favourable manufacture, there is a constant need to develop novel crop treatment compositions which have advantages over those known, at least in some areas.

It was therefore an object of the present invention to provide further compounds which increase tolerance to abiotic stress in plants.

The present invention accordingly provides for the use of a compound having the formula (I)

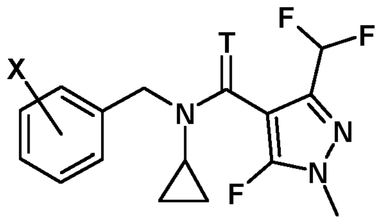

(I)

selected from the group consisting of:

N-cyclopropyl-N-(2-cyclopropylbenzyl)-3-(difluoromethyl)-5-fluoro-1-methyl-1H-pyrazole-4-carboxamide (compound A2),
N-(2-tert-butylbenzyl)-N-cyclopropyl-3-(difluoromethyl)-5-fluoro-1-methyl-1H-pyrazole-4-carboxamide (compound A3),
N-(5-chloro-2-ethylbenzyl)-N-cyclopropyl-3-(difluoromethyl)-5-fluoro-1-methyl-1H-pyrazole-4-carboxamide (compound A4),
N-(5-chloro-2-isopropylbenzyl)-N-cyclopropyl-3-(difluoromethyl)-5-fluoro-1-methyl-1H-pyrazole-4-carboxamide (compound A5),
N-cyclopropyl-3-(difluoromethyl)-N-(2-ethyl-5-fluorobenzyl)-5-fluoro-1-methyl-1H-pyrazole-4-carboxamide (compound A6),
N-cyclopropyl-3-(difluoromethyl)-5-fluoro-N-(5-fluoro-2-isopropylbenzyl)-1-methyl-1H-pyrazole-4-carboxamide (compound A7),
N-cyclopropyl-N-(2-cyclopropyl-5-fluorobenzyl)-3-(difluoromethyl)-5-fluoro-1-methyl-1H-pyrazole-4-carboxamide (compound A8),
N-cyclopropyl-3-(difluoromethyl)-N-(2-ethyl-5-methylbenzyl)-5-fluoro-1-methyl-1H-pyrazole-4-carboxamide (compound A11),
N-cyclopropyl-3-(difluoromethyl)-5-fluoro-N-(2-isopropyl-5-methylbenzyl)-1-methyl-1H-pyrazole-4-carboxamide (compound A12),
N-(2-tert-butyl-5-methylbenzyl)-N-cyclopropyl-3-(difluoromethyl)-5-fluoro-1-methyl-1H-pyrazole-4-carboxamide (compound A14),
N-cyclopropyl-3-(difluoromethyl)-5-fluoro-1-methyl-N-[5-methyl-2-(trifluoromethyl)benzyl]-1H-pyrazole-4-carboxamide (compound A16),
N-cyclopropyl-3-(difluoromethyl)-N-(2-ethyl-4,5-dimethylbenzyl)-5-fluoro-1-methyl-1H-pyrazole-4-carboxamide (compound A19), and
N-cyclopropyl-3-(difluoromethyl)-5-fluoro-N-(2-isopropylbenzyl)-1-methyl-1H-pyrazole-4-carbothio-amide (compound A20);
or an agrochemically acceptable salt thereof,
for increasing the drought tolerance of plants which are without fungal infection and without infection pressure, wherein said compound is applied to said plants or the locus in which they grow at an application rate of from 0.001 kg/ha to 2 kg/ha or as seed treatment at an application rate of from 0.001 to 250 g/kg of seeds.

The invention further relates to a method for increasing the drought tolerance of plants which are without fungal

infection and without infection pressure, comprising applying to said plants, to the seeds from which they grow or to the locus in which they grow, a non-phytotoxic, effective for enhancing the resistance of plants to drought, amount of a compound according to formula (I)

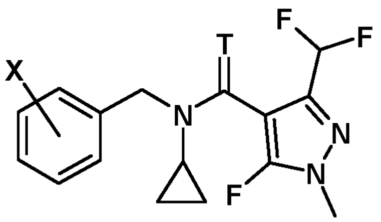

(I)

selected from the group consisting of :

N-cyclopropyl-N-(2-cyclopropylbenzyl)-3-(difluoromethyl)-5-fluoro-1-methyl-1H-pyrazole-4-carboxamide (compound A2),
N-(2-tert-butylbenzyl)-N-cyclopropyl-3-(difluoromethyl)-5-fluoro-1-methyl-1H-pyrazole-4-carboxamide (compound A3),
N-(5-chloro-2-ethylbenzyl)-N-cyclopropyl-3-(difluoromethyl)-5-fluoro-1-methyl-1H-pyrazole-4-carboxamide (compound A4),
N-(5-chloro-2-isopropylbenzyl)-N-cyclopropyl-3-(difluoromethyl)-5-fluoro-1-methyl-1H-pyrazole-4-carboxamide (compound A5),
N-cyclopropyl-3-(difluoromethyl)-N-(2-ethyl-5-fluorobenzyl)-5-fluoro-1-methyl-1H-pyrazole-4-carboxamide (compound A6),
N-cyclopropyl-3-(difluoromethyl)-5-fluoro-N-(5-fluoro-2-isopropylbenzyl)-1-methyl-1H-pyrazole-4-carboxamide (compound A7),
N-cyclopropyl-N-(2-cyclopropyl-5-fluorobenzyl)-3-(difluoromethyl)-5-fluoro-1-methyl-1H-pyrazole-4-carboxamide (compound A8),
N-cyclopropyl-3-(difluoromethyl)-N-(2-ethyl-5-methylbenzyl)-5-fluoro-1-methyl-1H-pyrazole-4-carboxamide (compound A11),
N-cyclopropyl-3-(difluoromethyl)-5-fluoro-N-(2-isopropyl-5-methylbenzyl)-1-methyl-1H-pyrazole-4-carboxamide (compound A12),
N-(2-tert-butyl-5-methylbenzyl)-N-cyclopropyl-3-(difluoromethyl)-5-fluoro-1-methyl-1H-pyrazole-4-carboxamide (compound A14),
N-cyclopropyl-3-(difluoromethyl)-5-fluoro-1-methyl-N-[5-methyl-2-(trifluoromethyl)benzyl]-1H-pyrazole-4-carboxamide (compound A16),
N-cyclopropyl-3-(difluoromethyl)-N-(2-ethyl-4,5-dimethylbenzyl)-5-fluoro-1-methyl-1H-pyrazole-4-carboxamide (compound A19), and
N-cyclopropyl-3-(difluoromethyl)-5-fluoro-N-(2-isopropylbenzyl)-1-methyl-1H-pyrazole-4-carbothio-amide (compound A20);
or an agrochemically acceptable salt thereof,
wherein said compound is applied to said plants or the locus in which they grow at an application rate of from 0.001 kg/ha to 2 kg/ha or as seed treatment at an application rate of from 0.001 to 250 g/kg of seeds.

Preference is given to the use and method according to the invention wherein the compound of the formula (I) is _N-(5-chloro-2-isopropylbenzyl)-N-cyclopropyl-3-(difluoromethyl)-5-fluoro-1-methyl-1H-pyrazole-4-carboxamide (compound A5),
or an agrochemically acceptable salt thereof.

The term "useful plants" as used here refers to crop plants which are employed as plants for obtaining foods, animal feeds or for industrial purposes.

The present invention accordingly provides for the use of at least one compound of formula (I) as herein defined or salts thereof, and of any desired mixtures of compound of formula (I) as herein defined or salts thereof with active agrochemical ingredients in accordance with the definition below, and to method using said compound, mixtures or salts, for enhancing the resistance of plants to abiotic stress factors, preferably to drought stress, especially for enhancing plant growth and/or for increasing plant yield.

The present invention further provides a spray solution for treatment of plants, comprising an amount, effective for enhancing the resistance of plants to abiotic stress factors, preferably to drought stress, of at least one compound

of formula (I) as herein defined or salts thereof. The abiotic stress conditions which can be relativized may include, for example, drought, cold and hot conditions, osmotic stress, waterlogging, elevated soil salinity, elevated exposure to minerals, ozone conditions, strong light conditions, limited availability of nitrogen nutrients, limited availability of phosphorus nutrients.

In one embodiment, it is possible, for example, that at least one compound of formula (I) as herein defined or salts thereof is applied by spray application to appropriate plants or plant parts to be treated. The use of at least one compound of formula (I) as herein defined or salts thereof envisaged in accordance with the invention is effected preferably with a dosage between 0.001 and 2 kg/ha, especially preferably between 0.005 and 1 kg/ha. When, in the context of the present invention, abscisic acid is used simultaneously with at least one compound of formula (I) as herein defined or salts thereof, for example in the context of a joint preparation or formulation, abscisic acid is preferably added in a dosage between 0.001 and 3 kg/ha, more preferably between 0.005 and 2 kg/ha, especially preferably between 0.01 and 1 kg/ha.

The term "resistance to abiotic stress" is understood in the context of the present invention to mean various kinds of advantages for plants. Such advantageous properties are manifested, for example, in the following improved plant characteristics: improved root growth with regard to surface area and depth, increased stolon and tiller formation, stronger and more productive stolons and tillers, improvement in shoot growth, increased lodging resistance, increased shoot base diameter, increased leaf area, higher yields of nutrients and constituents, for example carbohydrates, fats, oils, proteins, vitamins, minerals, essential oils, dyes, fibers, better fiber quality, earlier flowering, increased number of flowers, reduced content of toxic products such as mycotoxins, reduced content of residues or disadvantageous constituents of any kind, or better digestibility, improved storage stability of the harvested material, improved tolerance to disadvantageous temperatures, improved tolerance to drought and aridity or lack of water (though aridity and lack of water similarly cause drought stress), and also oxygen deficiency as a result of waterlogging, improved tolerance to elevated salt contents in soil and water, enhanced tolerance to ozone stress, improved compatibility with respect to herbicides and other crop treatment compositions, improved water absorption and photosynthesis performance, advantageous plant properties, for example acceleration of ripening, more homogeneous ripening, greater attractiveness to beneficial animals, improved pollination, or other advantages well known to a person skilled in the art.

More particularly, the inventive use and method exhibits the advantages described in spray application to plants and plant parts. Combinations of at least one compound of formula (I) as herein defined or salts thereof with substances including insecticides, attractants, acaricides, fungicides, nematicides, herbicides, growth regulators, safeners, substances which influence plant maturity, and bactericides can likewise be employed in the control of plant disorders in the context of the present invention. In addition, the combined use of at least one compound of formula (I) as herein defined or salts thereof with genetically modified cultivars with a view to increased tolerance to abiotic stress, preferably drought stress, is likewise possible.

The following list of fungicides in combination with which the compounds according to the invention can be used is intended to illustrate the possible combinations, but not to impose any limitation:
The active ingredients specified herein by their "common name" are known and described, for example, in the Pesticide Manual or can be searched in the internet (e.g. http://www.alanwood.net/pesticides).

Where a compound (A) or a compound (B) can be present in tautomeric form, such a compound is understood hereinabove and herein below also to include, where applicable, corresponding tautomeric forms, even when these are not specifically mentioned in each case.

1) Inhibitors of the ergosterol biosynthesis, for example (1.1) aldimorph, (1.2) azaconazole, (1.3) bitertanol, (1.4) bromuconazole, (1.5) cyproconazole, (1.6) diclobutrazole, (1.7) difenoconazole, (1.8) diniconazole, (1.9) diniconazole-M, (1.10) dodemorph, (1.11) dodemorph acetate, (1.12) epoxiconazole, (1.13) etaconazole, (1.14) fenarimol, (1.15) fenbuconazole, (1.16) fenhexamid, (1.17) fenpropidin, (1.18) fenpropimorph, (1.19) fluquinconazole, (1.20) flurprimidol, (1.21) flusilazole, (1.22) flutriafol, (1.23) furconazole, (1.24) furconazole-cis, (1.25) hexaconazole, (1.26) imazalil, (1.27) imazalil sulfate, (1.28) imibenconazole, (1.29) ipconazole, (1.30) metconazole, (1.31) myclobutanil, (1.32) naftifine, (1.33) nuarimol, (1.34) oxpoconazole, (1.35) paclobutrazol, (1.36) pefurazoate, (1.37) penconazole, (1.38) piperalin, (1.39) prochloraz, (1.40) propiconazole, (1.41) prothioconazole, (1.42) pyributicarb, (1.43) pyrifenox, (1.44) quinconazole, (1.45) simeconazole, (1.46) spiroxamine, (1.47) tebuconazole, (1.48) terbinafine, (1.49) tetraconazole, (1.50) triadimefon, (1.51) triadimenol, (1.52) tridemorph, (1.53) triflumizole, (1.54) triforine, (1.55) triticonazole, (1.56) uniconazole, (1.57) uniconazole-p, (1.58) viniconazole, (1.59) voriconazole, (1.60) 1-(4-chlorophenyl)-2-(1H-1,2,4-triazol-1-yl)cycloheptanol, (1.61) methyl 1-(2,2-dimethyl-2,3 -dihydro- 1H-inden-1-yl)-1H-imidazole-5-carboxylate, (1.62) N'-{5-(difluoromethyl)-2-methyl-4-[3-(trimethylsilyl)propoxy]phenyl}-N-ethyl-N-methylimidoformamide, (1.63) N-ethyl-N-methyl-N'-{2-methyl-5-(trifluoromethyl)-4-[3-(trimethylsilyl)propoxy]phenyl}imidoformamide, (1.64) O-[1-(4-methoxyphenoxy)-3,3-dimethylbutan-2-yl] 1H-imidazole-1-carbothioate, (1.65) Pyrisoxazole.

2) Inhibitors of the respiratory chain at complex I or II, for example (2.1) bixafen, (2.2) boscalid, (2.3) carboxin, (2.4)

diflumetorim, (2.5) fenfuram, (2.6) fluopyram, (2.7) flutolanil, (2.8) fluxapyroxad, (2.9) furametpyr, (2.10) furmecyclox, (2.11) isopyrazam (mixture of syn-epimeric racemate 1RS,4SR,9RS and anti-epimeric racemate 1RS,4SR,9SR), (2.12) isopyrazam (anti-epimeric racemate 1RS,4SR,9SR), (2.13) isopyrazam (anti-epimeric enantiomer 1R,4S,9S), (2.14) isopyrazam (anti-epimeric enantiomer 1S,4R,9R), (2.15) isopyrazam (syn epimeric racemate 1RS,4SR,9RS), (2.16) isopyrazam (syn-epimeric enantiomer 1R,4S,9R), (2.17) isopyrazam (syn-epimeric enantiomer 1S,4R,9S), (2.18) mepronil, (2.19) oxycarboxin, (2.20) penflufen, (2.21) penthiopyrad, (2.22) sedaxane, (2.23) thifluzamide, (2.24) 1-methyl-N-[2-(1,1,2,2-tetrafluoroethoxy)phenyl]-3-(trifluoromethyl)-1H-pyrazole-4-carboxamide, (2.25) 3-(difluoromethyl)-1-methyl-N-[2-(1,1,2,2-tetrafluoroethoxy)phenyl]-1H-pyrazole-4-carboxamide, (2.26) 3-(difluoromethyl)-N-[4-fluoro-2-(1,1,2,3,3,3-hexafluoropropoxy)phenyl]-1-methyl-1H-pyrazole-4-carboxamide, (2.27) N-[1-(2,4-dichlorophenyl)-1-methoxypropan-2-yl]-3-(difluoromethyl)-1-methyl-1H-pyrazole-4-carboxamide, (2.28)5,8-difluoro-N-[2-(2-fluoro-4-{[4-(trifluoromethyl)pyridin-2-yl]oxy}phenyl)ethyl]quinazolin-4-amine, (2.29) benzovindiflupyr, (2.30) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.31) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.32) 3-(difluoromethyl)-1-methyl-N-(1,1,3-trimethyl-2,3-dihydro-1H-inden-4-yl)-1H-pyrazole-4-carboxamide, (2.33) 1,3,5-trimethyl-N-(1,1,3-trimethyl-2,3-dihydro-1H-inden-4-yl)-1H-pyrazole-4-carboxamide, (2.34) 1-methyl-3-(trifluoromethyl)-N-(1,1,3-trimethyl-2,3-dihydro-1H-inden-4-yl)-1H-pyrazole-4-carboxamide, (2.35) 1-methyl-3-(trifluoromethyl)-N-[(3R)-1,1,3-trimethyl-2,3-dihydro-1H-inden-4-yl]-1H-pyrazole-4-carboxamide, (2.36) 1-methyl-3-(trifluoromethyl)-N-[(3S)-1,1,3-trimethyl-2,3-dihydro-1H-inden-4-yl]-1H-pyrazole-4-carboxamide, (2.37) 3-(difhioromethyl)-1-methyl-N-[(3S)-1,1,3-trimethyl-2,3-dihydro-1H-inden-4-yl]-1H-pyrazole-4-carboxamide, (2.38) 3-(difluoromethyl)-1-methyl-N-[(3R)-1,1,3-trimethyl-2,3-dihydro-1H-inden-4-yl]-1H-pyrazole-4-carboxamide, (2.39) 1,3,5-trimethyl-N-[(3R)-1,1,3-trimethyl-2,3-dihydro-1H-inden-4-yl]-1H-pyrazole-4-carboxamide, (2.40) 1,3,5-trimethyl-N-[(3S)-1,1,3-trimethyl-2,3-dihydro-1H-inden-4-yl]-1H-pyrazole-4-carboxamide, (2.41) benodanil, (2.42) 2-chloro-N-(1,1,3-trimethyl-2,3-dihydro-1H-inden-4-yl)pyridine-3-carboxamide, (2.43) Isofetamid.

3) Inhibitors of the respiratory chain at complex III, for example (3.1) ametoctradin, (3.2) amisulbrom, (3.3) azoxystrobin, (3.4) cyazofamid, (3.5) coumethoxystrobin, (3.6) coumoxystrobin, (3.7) dimoxystrobin, (3.8) enoxastrobin, (3.9) famoxadone, (3.10) fenamidone, (3.11) flufenoxystrobin, (3.12) fluoxastrobin, (3.13) kresoxim-methyl, (3.14) metominostrobin, (3.15) orysastrobin, (3.16) picoxystrobin, (3.17) pyraclostrobin, (3.18) pyrametostrobin, (3.19) pyraoxystrobin, (3.20) pyribencarb, (3.21) triclopyricarb, (3.22) trifloxystrobin, (3.23) (2E)-2-(2-{[6-(3-chloro-2-methylphenoxy)-5-fluoropyrimidin-4-yl]oxy}phenyl)-2-(methoxyimino)-N-methylacetamide, (3.24) (2E)-2-(methoxyimino)-N-methyl-2-(2-{[({(1E)-1-[3-(trifluoromethyl)phenyl]ethylidene}amino)oxy]methyl}phenyl)acetamide, (3.25) (2E)-2-(methoxyimino)-N-methyl-2-{2-[(E)-({1-[3-(trifluoromethyl)phenyl]ethoxy}imino)methyl]phenyl}acetamide, (3.26) (2E)-2- {2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.27) Fenaminostrobin, (3.28) 5-methoxy-2-methyl-4-(2-{[({(1E)-1-[3-(trifluoromethyl)phenyl]ethylidene}amino)oxy]methyl}phenyl)-2,4-dihydro-3H-1,2,4-triazol-3-one, (3.29) methyl (2E)-2-{2-[({cyclopropyl[(4-methoxyphenyl)imino]methyl}sulfanyl)methyl]phenyl}-3-methoxyacrylate, (3.30) N-(3-ethyl-3,5,5-trimethylcyclohexyl)-3-formamido-2-hydroxybenzamide, (3.31) 2-{2-[(2,5-dimethylphenoxy)methyl]phenyl}-2-methoxy-N-methylacetamide, (3.32) 2-{2-[(2,5-dimethylphenoxy)methyl]phenyl}-2-methoxy-N-methylacetamide; (3.33) (2E,3Z)-5-{[1-(4-chlorophenyl)-1H-pyrazol-3-yl]oxy}-2-(methoxyimino)-N,3-dimethylpent-3-enamide.

4) Inhibitors of the mitosis and cell division, for example (4.1) benomyl, (4.2) carbendazim, (4.3) chlorfenazole, (4.4) diethofencarb, (4.5) ethaboxam, (4.6) fluopicolide, (4.7) fuberidazole, (4.8) pencycuron, (4.9) thiabendazole, (4.10) thiophanate-methyl, (4.11) thiophanate, (4.12) zoxamide, (4.13) 5-chloro-7-(4-methylpiperidin-1-yl)-6-(2,4,6-trifluorophenyl)[1,2,4]triazolo[1,5-a]pyrimidine, (4.14) 3-chloro-5-(6-chloropyridin-3-yl)-6-methyl-4-(2,4,6-trifluorophenyl)pyridazine.

5) Compounds capable to have a multisite action, for example (5.1) bordeaux mixture, (5.2) captafol, (5.3) captan, (5.4) chlorothalonil, (5.5) copper hydroxide, (5.6) copper naphthenate, (5.7) copper oxide, (5.8) copper oxychloride, (5.9) copper(2+) sulfate, (5.10) dichlofluanid, (5.11) dithianon, (5.12) dodine, (5.13) dodine free base, (5.14) ferbam, (5.15) fluorofolpet, (5.16) folpet, (5.17) guazatine, (5.18) guazatine acetate, (5.19) iminoctadine, (5.20) iminoctadine albesilate, (5.21) iminoctadine triacetate, (5.22) mancopper, (5.23) mancozeb, (5.24) maneb, (5.25) metiram, (5.26) metiram zinc, (5.27) oxine-copper, (5.28) propamidine, (5.29) propineb, (5.30) sulfur and sulfur preparations including calcium polysulfide, (5.31) thiram, (5.32) tolylfluanid, (5.33) zineb, (5.34) ziram, (5.35) anilazine.

6) Compounds capable to induce a host defence, for example (6.1) acibenzolar-S-methyl, (6.2) isotianil, (6.3) probenazole, (6.4) tiadinil, (6.5) laminarin.

7) Inhibitors of the amino acid and/or protein biosynthesis, for example (7.1) andoprim, (7.2) blasticidin-S, (7.3) cyprodinil, (7.4) kasugamycin, (7.5) kasugamycin hydrochloride hydrate, (7.6) mepanipyrim, (7.7) pyrimethanil, (7.8) 3-(5-fluoro-3,3,4,4-tetramethyl-3,4-dihydroisoquinolin-1-yl)quinoline, (7.9) oxytetracycline, (7.10) streptomycin.

8) Inhibitors of the ATP production, for example (8.1) fentin acetate, (8.2) fentin chloride, (8.3) fentin hydroxide, (8.4) silthiofam.

9) Inhibitors of the cell wall synthesis, for example (9.1) benthiavalicarb, (9.2) dimethomorph, (9.3) flumorph, (9.4) iprovalicarb, (9.5) mandipropamid, (9.6) polyoxins, (9.7) polyoxorim, (9.8) validamycin A, (9.9) valifenalate, (9.10) polyoxin B.

10) Inhibitors of the lipid and membrane synthesis, for example (10.1) biphenyl, (10.2) chloroneb, (10.3) dicloran, (10.4) edifenphos, (10.5) etridiazole, (10.6) iodocarb, (10.7) iprobenfos, (10.8) isoprothiolane, (10.9) propamocarb, (10.10) propamocarb hydrochloride, (10.11) prothiocarb, (10.12) pyrazophos, (10.13) quintozene, (10.14) tecnazene, (10.15) tolclofos-methyl.

11) Inhibitors of the melanin biosynthesis, for example (11.1) carpropamid, (11.2) diclocymet, (11.3) fenoxanil, (11.4) phthalide, (11.5) pyroquilon, (11.6) tricyclazole, (11.7) 2,2,2-trifluoroethyl {3-methyl-1-[(4-methylbenzoyl)amino]butan-2-yl}carbamate.

12) Inhibitors of the nucleic acid synthesis, for example (12.1) benalaxyl, (12.2) benalaxyl-M (kiralaxyl), (12.3) bupirimate, (12.4) clozylacon, (12.5) dimethirimol, (12.6) ethirimol, (12.7) furalaxyl, (12.8) hymexazol, (12.9) metalaxyl, (12.10) metalaxyl-M (mefenoxam), (12.11) ofurace, (12.12) oxadixyl, (12.13) oxolinic acid, (12.14) octhilinone.

13) Inhibitors of the signal transduction, for example (13.1) chlozolinate, (13.2) fenpiclonil, (13.3) fludioxonil, (13.4) iprodione, (13.5) procymidone, (13.6) quinoxyfen, (13.7) vinclozolin, (13.8) proquinazid.

14) Compounds capable to act as an uncoupler, for example (14.1) binapacryl, (14.2) dinocap, (14.3) ferimzone, (14.4) fluazinam, (14.5) meptyldinocap.

15) Further compounds, for example (15.1) benthiazole, (15.2) bethoxazin, (15.3) capsimycin, (15.4) carvone, (15.5) chinomethionat, (15.6) pyriofenone (chlazafenone), (15.7) cufraneb, (15.8) cyflufenamid, (15.9) cymoxanil, (15.10) cyprosulfamide, (15.11) dazomet, (15.12) debacarb, (15.13) dichlorophen, (15.14) diclomezine, (15.15) difenzoquat, (15.16) difenzoquat metilsulfate, (15.17) diphenylamine, (15.18) ecomate, (15.19) fenpyrazamine, (15.20) flumetover, (15.21) fluoroimide, (15.22) flusulfamide, (15.23) flutianil, (15.24) fosetyl-aluminium, (15.25) fosetyl-calcium, (15.26) fosetyl-sodium, (15.27) hexachlorobenzene, (15.28) irumamycin, (15.29) methasulfocarb, (15.30) methyl isothiocyanate, (15.31) metrafenone, (15.32) mildiomycin, (15.33) natamycin, (15.34) nickel dimethyldithiocarbamate, (15.35) nitrothal-isopropyl, (15.37) oxamocarb, (15.38) oxyfenthiin, (15.39) pentachlorophenol and salts, (15.40) phenothrin, (15.41) phosphorous acid and its salts, (15.42) propamocarb-fosetylate, (15.43) propanosine-sodium, (15.44) pyrimorph, (15.45) (2E)-3-(4-tert-butylphenyl)-3-(2-chloropyridin-4-yl)-1-(morpholin-4-yl)prop-2-en-1-one, (15.46) (2Z)-3 -(4-tert-butylphenyl)-3-(2-chloropyridin-4-yl)-1-(morpholin-4-yl)prop-2-en-1-one, (15.47) pyrrolnitrine, (15.48) tebufloquin, (15.49) tecloftalam, (15.50) tolnifanide, (15.51) triazoxide, (15.52) trichlamide, (15.53) zarilamid, (15.54) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (15.55) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.56) 1-(4-{4-[(5S)-5-(2,6-difluorophenyl)-4,5 -dihydro-1,2-oxazol-3-yl]-1,3-thiazol-2-yl}piperidin-1-yl)-2-[5 -methyl-3-(trifluoromethyl)-1H-pyrazol-1-yl]ethanone, (15.57) 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.58) 1-(4-methoxyphenoxy)-3,3-dimethylbutan-2-yl 1H-imidazole-1-carboxylate, (15.59) 2,3,5,6-tetrachloro-4-(methylsulfonyl)pyridine, (15.60) 2,3-dibutyl-6-chlorothieno[2,3-d]pyrimidin-4(3H)-one, (15.61) 2,6-dimethyl-1H,5H-[1,4]dithiino[2,3-c:5,6-c']dipyrrole-1,3,5,7(2H,6H)-tetrone, (15.62) 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone, (15.63) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone, (15.64) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone, (15.65) 2-butoxy-6-iodo-3-propyl-4H-chromen-4-one, (15.66) 2-chloro-5-[2-chloro-1-(2,6-difluoro-4-methoxyphenyl)-4-methyl-1H-imidazol-5-yl]pyridine, (15.67) 2-phenylphenol and salts, (15.68) 3-(4,4,5-trifluoro-3,3-dimethyl-3,4-dihydroisoquinolin-1-yl)quinoline, (15.69) 3,4,5-trichloropyridine-2,6-dicarbonitrile, (15.70) 3-chloro-5-(4-chlorophenyl)-4-(2,6-difluorophenyl)-6-methylpyridazine, (15.71) 4-(4-chlorophenyl)-5-(2,6-difluorophenyl)-3,6-dimethylpyridazine, (15.72) 5 -amino-1,3,4-thiadiazole-2-thiol, (15.73) 5-chloro-N'-phenyl-N'-(prop-2-yn-1-yl)thiophene-2-sulfonohydrazide, (15.74) 5-fluoro-2-[(4-fluorobenzyl)oxy]pyrimidin-4-amine, (15.75) 5-fluoro-2-[(4-methylbenzyl)oxy]pyrimidin-4-amine, (15.76) 5-methyl-6-octyl[1,2,4]triazolo[1,5-a]pyrimidin-7-amine, (15.77) ethyl (2Z)-3-amino-2-cyano-3-phenylacrylate, (15.78) N'-(4-{[3-(4-chlorobenzyl)-1,2,4-thiadiazol-5-yl]oxy}-2,5-dimethylphenyl)-N-ethyl-N-methylimidoformamide, (15.79) N-(4-chlorobenzyl)-3-[3-methoxy-4-(prop-2-yn-1-yloxy)phenyl]propanamide, (15.80) N-[(4-chlorophenyl)(cyano)methyl]-3-[3-methoxy-4-(prop-2-yn-1-yloxy)phenyl]propanamide, (15.81) N-[(5-bromo-3-chloropyridin-2-yl)methyl]-2,4-dichloronicotinamide, (15.82) N-[1-(5-bromo-3-chloropyridin-2-yl)ethyl]-2,4-dichloronicotinamide, (15.83) N-[1-(5-bromo-3-chloropyridin-2-yl)ethyl]-2-fluoro-4-iodonicotinamide, (15.84) N-{(E)-[(cyclopropylmethoxy)imino][6-(difluoromethoxy)-2,3-difluorophenyl]methyl}-2-phenylacetamide, (15.85) N-{(Z)-[(cyclopropylmethoxy)imino][6-(difluoromethoxy)-2,3-difluorophenyl]methyl}-2-phenylacetamide, (15.86) N'-{4-[(3-tert-butyl-4-cyano-1,2-thiazol-5-yl)oxy]-2-chloro-5-methylphenyl}-N-ethyl-N-methylimidoformamide, (15.87) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, (15.88) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide, (15.89) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide, (15.90) pentyl {6-[({[(1-methyl-1H-tetrazol-5-yl)(phenyl)methylene]amino}oxy)methyl]pyridin-2-yl}carbamate, (15.91) phenazine-1-carboxylic acid, (15.92) quinolin-8-ol, (15.93) quinolin-8-ol sulfate (2:1), (15.94) tert-butyl {6-[({[(1-methyl-1H-tetrazol-5-yl)(phenyl)methylene]amino}oxy)methyl]pyridin-2-yl}carbamate, (15.95) 1-methyl-3-(trifluoromethyl)-N-[2'-(trifluoromethyl)biphenyl-2-yl]-1H-pyrazole-4-carboxamide, (15.96) N-(4'-chlorobiphenyl-2-yl)-3-(difluoromethyl)-1-methyl-1H-pyrazole-4-carboxamide, (15.97) N-(2',4'-dichlorobiphenyl-2-yl)-3-(difluoromethyl)-1-methyl-1H-pyrazole-4-carboxamide, (15.98) 3-(difluoromethyl)-1-methyl-N-[4'-(trifluoromethyl)biphenyl-2-yl]-1H-pyrazole-4-carboxamide, (15.99) N-(2',5'-difluorobiphenyl-2-yl)-1-methyl-3-(trifluoromethyl)-1H-pyrazole-4-carboxamide, (15.100) 3-(difluoromethyl)-1-methyl-N-[4'-(prop-1-yn-1-yl)biphenyl-2-yl]-1H-pyrazole-4-carboxamide, (15.101) 5-fluoro-1,3-dimethyl-N-[4'-(prop-1-yn-1-yl)biphenyl-2-yl]-1H-pyrazole-4-carboxamide, (15.102) 2-chloro-N-[4'-(prop-1-yn-1-yl)biphenyl-2-yl]nicotinamide, (15.103) 3-(difluoromethyl)-N-[4'-(3,3-dimethylbut-1-yn-1-yl)biphenyl-2-yl]-1-methyl-1H-pyrazole-4-carboxamide, (15.104) N-[4'-(3,3-dimethylbut-1-yn-1-yl)biphenyl-2-yl]-5-fluoro-1,3-dimethyl-1H-pyrazole-4-carboxamide, (15.105) 3-(difluoromethyl)-N-(4'-ethynylbiphenyl-2-yl)-1-methyl-1H-pyrazole-4-carboxamide, (15.106) N-(4'-ethynylbiphenyl-2-yl)-5-fluoro-1,3-dimethyl-1H-pyrazole-4-carboxamide, (15.107) 2-chloro-N-(4'-ethynylbiphenyl-2-yl)nicotinamide, (15.108) 2-chloro-N-[4'-(3,3-dimethylbut-1-yn-1-yl)biphenyl-2-yl]nicotinamide, (15.109) 4-(difluoromethyl)-2-methyl-N-[4'-(trifluoromethyl)biphenyl-2-yl]-1,3-thiazole-5-carboxamide, (15.110) 5-fluoro-N-[4'-(3-hydroxy-3-methylbut-1-yn-1-yl)biphenyl-2-yl]-1,3-dimethyl-1H-pyrazole-4-carboxamide, (15.111) 2-chloro-N-[4'-(3-hydroxy-3-methylbut-1-yn-1-yl)biphenyl-2-yl]nicotinamide, (15.112) 3-(difluoromethyl)-N-[4'-(3-methoxy-3-methylbut-1-yn-1-yl)biphenyl-2-yl]-1-methyl-1H-pyrazole-4-carboxamide, (15.113) 5-fluoro-N-[4'-(3-methoxy-3-methylbut-1-yn-1-yl)biphenyl-2-yl]-1,3-dimethyl-1H-pyrazole-4-carboxamide, (15.114) 2-chloro-N-[4'-(3-methoxy-3-methylbut-1-yn-1-yl)biphenyl-2-yl]nicotinamide, (15.115) (5-bromo-2-methoxy-4-methylpyridin-3-yl)(2,3,4-trimethoxy-6-methylphenyl)methanone, (15.116) N-[2-(4-{[3-(4-chlorophenyl)prop-2-yn-1-yl]oxy}-3-methoxyphenyl)ethyl]-N2-(methylsulfonyl)valinamide, (15.117) 4-oxo-4-[(2-phenylethyl)amino]butanoic acid, (15.118) but-3-yn-1-yl {6-[({[(Z)-(1-methyl-1H-tetrazol-5-yl)(phenyl)methylene]amino}oxy)methyl]pyridin-2-yl}carbamate, (15.119) 4-amino-5-fluoropyrimidin-2-ol (tautomeric form: 4-amino-5-fluoropyrimidin-2(1H)-one), (15.120) propyl 3,4,5-trihydroxybenzoate, (15.121) 1,3-dimethyl-N-(1,1,3-trimethyl-2,3-dihydro-1H-inden-4-yl)-1H-pyrazole-4-carboxamide, (15.122) 1,3-dimethyl-N-[(3R)-1,1,3-trimethyl-2,3-dihydro-1H-inden-4-yl]-1H-pyrazole-4-carboxamide, (15.123) 1,3-dimethyl-N-[(3S)-1,1,3-trimethyl-2,3-dihydro-1H-inden-4-yl]-1H-pyrazole-4-carboxamide, (15.124) [3-(4-chloro-2-fluorophenyl)-5-(2,4-difluorophenyl)-1,2-oxazol-4-yl](pyridin-3-yl)methanol, (15.125) (S)-[3-(4-chloro-2-fluorophenyl)-5-(2,4-difluorophenyl)-1,2-oxazol-4-yl] (pyridin-3-yl)methanol, (15.126) (R)-[3 -(4-chloro-2-fluorophenyl)-5 - (2,4-difluorophenyl)-1,2-oxazol-4-yl] (pyridin-3-yl)methanol, (15.127) 2-{[3-(2-chlorophenyl)-2-(2,4-difluorophenyl)oxiran-2-yl]methyl}-2,4-dihydro-3H-1,2,4-triazole-3-thione, (15.128) 1-{[3-(2-chlorophenyl)-2-(2,4-difluorophenyl)oxiran-2-yl]methyl}-1H-1,2,4-triazol-5-yl thiocyanate, (15.129) 5-(allylsulfanyl)-1-{[3-(2-chlorophenyl)-2-(2,4-difluorophenyl)oxiran-2-yl]methyl}-1H-1,2,4-triazole, (15.130) 2-[1-(2,4-dichlorophenyl)-5-hydroxy-2,6,6-trimethylheptan-4-yl]-2,4-dihydro-3H-1,2,4-triazole-3-thione, (15.131) 2-{[rel(2R,3S)-3-(2-chlorophenyl)-2-(2,4-difluorophenyl)oxiran-2-yl]methyl}-2,4-dihydro-3H-1,2,4-triazole-3-thione, (15.132) 2-{[rel(2R,3R)-3-(2-chlorophenyl)-2-(2,4-difluorophenyl)oxiran-2-yl]methyl}-2,4-dihydro-3H-1,2,4-triazole-3-thione, (15.133) 1-{[rel(2R,3S)-3-(2-chlorophenyl)-2-(2,4-difluorophenyl)oxiran-2-yl]methyl}-1H-1,2,4-triazol-5-yl thiocyanate, (15.134) 1-{[rel(2R,3R)-3-(2-chlorophenyl)-2-(2,4-difluorophenyl)oxiran-2-yl]methyl}-1H-1,2,4-triazol-5-yl thiocyanate, (15.135) 5-(allylsulfanyl)-1-{[rel(2R,3S)-3-(2-chlorophenyl)-2-(2,4-difluorophenyl)oxiran-2-yl]methyl}-1H-1,2,4-triazole, (15.136) 5-(allylsulfanyl)-1-{[rel(2R,3R)-3-(2-chlorophenyl)-2-(2,4-difluorophenyl)oxiran-2-yl]methyl}-1H-1,2,4-triazole, (15.137) 2-[(2S,4S,5S)-1-(2,4-dichlorophenyl)-5-hydroxy-2,6,6-trimethylheptan-4-yl]-2,4-dihydro-3H-1,2,4-triazole-3-thione, (15.138) 2-[(2R,4S,5S)-1-(2,4-dichlorophenyl)-5-hydroxy-2,6,6-trimethylheptan-4-yl]-2,4-dihydro-3H-1,2,4-triazole-3-thione, (15.139) 2-[(2R,4R,5R)-1-(2,4-dichlorophenyl)-5-hydroxy-2,6,6-trimethylheptan-4-yl]-2,4-dihydro-3H-1,2,4-triazole-3-thione, (15.140) 2-[(2S,4R,5R)-1-(2,4-dichlorophenyl)-5-hydroxy-2,6,6-trimethylheptan-4-yl]-2,4-dihydro-3H-1,2,4-triazole-3-thione, (15.141) 2-[(2S,4S,5R)-1-(2,4-dichlorophenyl)-5-hydroxy-2,6,6-trimethylheptan-4-yl]-2,4-dihydro-3H-1,2,4-triazole-3-thione, (15.142) 2-[(2R,4S,5R)-1-(2,4-dichlorophenyl)-5-hydroxy-2,6,6-trimethylheptan-4-yl]-2,4-dihydro-3H-1,2,4-triazole-3-thione, (15.143) 2-[(2R,4R,5S)-1-(2,4-dichlorophenyl)-5-hydroxy-2,6,6-trimethylheptan-4-yl]-2,4-dihydro-3H-1,2,4-triazole-3-thione, (15.144) 2-[(2S,4R,5S)-1-(2,4-dichlorophenyl)-5-hydroxy-2,6,6-trimethylheptan-4-yl]-2,4-dihydro-3H-1,2,4-triazole-3-thione, (15.145) 2-fluoro-6-(trifluoromethyl)-N-(1,1,3-trimethyl-2,3-dihydro-1H-inden-4-yl)benzamide, (15.146) 2-(6-benzylpyridin-2-yl)quinazoline, (15.147) 2-[6-(3-fluoro-4-methoxyphenyl)-5-methylpyridin-2-yl]quinazoline, (15.148) 3-(4,4-difluoro-3,3-dimethyl-3,4-dihydroisoquinolin-1-yl)quinoline, (15.149) Abscisic acid, (15.150) 3-(difluoromethyl)-N-methoxy-1-methyl-N-[1-(2,4,6-trichlorophenyl)propan-2-yl]-1H-pyrazole-4-carboxamide, (15.151) N'-[5-bromo-6-(2,3-dihydro-1H-inden-2-yloxy)-2-methylpyridin-3-yl]-N-ethyl-N-methylimidoformamide, (15.152) N'-{5-bromo-6-[1-(3,5-difluorophenyl)ethoxy]-2-methylpyridin-3-yl}-N-ethyl-N-methylimidoformamide, (15.153) N'-{5-bromo-6-[(1R)-1-(3,5 -difluorophenyl)ethoxy]-2-methylpyridin-3-yl}-N-ethyl-N-methylimidoformamide, (15.154) N'-{5-bromo-6-[(1S)-1-(3,5-difluorophenyl)ethoxy]-2-methylpyridin-3-yl}-N-ethyl-N-methylimidoformamide, (15.155) N'-{5-bromo-6-[(cis-4-isopropylcyclohexyl)oxy]-2-methylpyridin-3-yl}-N-ethyl-N-methylimidoformamide, (15.156) N'-{5-bromo-6-[(trans-4-isopropylcyclohexyl)oxy]-2-methylpyridin-3-yl}-N-ethyl-N-methylimidoformamide, (15.157) N-cyclopropyl-3-(difluoromethyl)-5-fluoro-N-(2-isopropylbenzyl)-1-methyl-1H-pyrazole-4-carboxamide, (15.158) N-cyclopropyl-N-(2-cyclopropylbenzyl)-3-(difluoromethyl)-5-fluoro-1-methyl-1H-pyrazole-4-carboxamide, (15.159) N-(2-tert-butylbenzyl)-N-cyclopropyl-3-(difluoromethyl)-5-fluoro-1-methyl-1H-pyrazole-4-carboxamide, (15.160) N-(5-chloro-2-ethylbenzyl)-N-cyclopropyl-3-(difluoromethyl)-5-fluoro-1-methyl-1H-pyrazole-4-carboxamide, (15.161) N-(5-chloro-2-isopropylbenzyl)-N-cyclopropyl-3-(difluoromethyl)-5-fluoro-1-methyl-1H-pyrazole-4-carboxamide, (15.162) N-cyclopropyl-3-(difluoromethyl)-N-(2-ethyl-5-fluorobenzyl)-5-fluoro-1-methyl-1H-pyrazole-4-carboxamide, (15.163) N-cyclopropyl-3-(difluoromethyl)-5-fluoro-N-(5-fluoro-2-isopropylbenzyl)-1-methyl-1H-pyrazole-4-carboxamide, (15.164) N-cyclopropyl-N-(2-cyclopropyl-5-fluorobenzyl)-3-(difluoromethyl)-5-fluoro-1-methyl-1H-pyrazole-4-carboxamide, (15.165) N-(2-cyclopentyl-5-fluorobenzyl)-N-cyclopropyl-3-(difluoromethyl)-5-fluoro-1-methyl-1H-pyrazole-4-carboxamide, (15.166) N-cyclopropyl-3 - (difluoromethyl)-5-fluoro-N-(2-fluoro-6-isopropylbenzyl)-1-methyl-1H-pyrazole-4-carboxamide, (15.167) N-cyclopropyl-3-(difluoromethyl)-N-(2-ethyl-5-methylbenzyl)-5-fluoro-1-methyl-1H-pyrazole-4-carboxamide, (15.168) N-cyclopropyl-3-(difluoromethyl)-5-fluoro-N-(2-isopropyl-5-methylbenzyl)-1-methyl-1H-pyrazole-4-carboxamide, (15.169) N-cyclopropyl-N-(2-cyclopropyl-5-methylbenzyl)-3-(difluoromethyl)-5-fluoro-1-methyl-1H-pyrazole-4-carboxamide, (15.170) N-(2-tert-butyl-5-methylbenzyl)-N-cyclopropyl-3-(difluoromethyl)-5-fluoro-1-methyl-1H-pyrazole-4-carboxamide, (15.171) N-[5-chloro-2-(trifluoromethyl)benzyl]-N-cyclopropyl-3-(difluoromethyl)-5-fluoro-1-methyl-1H-pyrazole-4-carboxamide, (15.172) N-cyclopropyl-3-(difluoromethyl)-5-fluoro-1-methyl-N-[5-methyl-2-(trifluoromethyl)benzyl]-1H-pyrazole-4-carboxamide, (15.173) N-[2-chloro-6-(trifluoromethyl)benzyl]-N-cyclopropyl-3-(difluoromethyl)-5-fluoro-1-methyl-1H-pyrazole-4-carboxamide, (15.174) N-[3-chloro-2-fluoro-6-(trifluoromethyl)benzyl]-N-cyclopropyl-3-(difluoromethyl)-5-fluoro-1-methyl-1H-pyrazole-4-carboxamide, (15.175) N-cyclopropyl-3-(difluoromethyl)-N-(2-ethyl-4,5-dimethylbenzyl)-5-fluoro-1-methyl-1H-pyrazole-4-carboxamide, (15.176) N-cyclopropyl-3-(difluoromethyl)-5-fluoro-N-(2-isopropylbenzyl)-1-methyl-1H-pyrazole-4-carbothioamide, (15.177) 3-(difluoromethyl)-N-(7-fluoro-1,1,3-trimethyl-2,3-dihydro-1H-inden-4-yl)-1-methyl-1H-pyrazole-4-carboxamide, (15.178) 3-(difluoromethyl)-N-[(3R)-7-fluoro-1,1,3-trimethyl-2,3-dihydro-1H-inden-4-yl]-1-methyl-1H-pyrazole-4-carboxamide, (15.179) 3-(difluoromethyl)-N-[(3S)-7-fluoro-1,1,3-trimethyl-2,3-dihydro-1H-inden-4-yl]-1-methyl-1H-pyrazole-4-carboxamide, (15.180) N'-(2,5-dimethyl-4-phenoxyphenyl)-N-ethyl-N-methylimidoformamide, (15.181) N'-{4-[(4,5-dichloro-1,3-thiazol-2-yl)oxy]-2,5-dimethylphenyl}-N-ethyl-N-methylimidoformamide, (15.182) N-(4-chloro-2,6-difluorophenyl)-4-(2-chloro-4-fluorophenyl)-1,3-dimethyl-1H-pyrazol-5-amine; (15.183) 2-[4-(4-chlorophenoxy)-2-(trifluoromethyl)phenyl]-1-(1H-1,2,4-triazol-1-yl)propan-2-ol, (15.184) 2-[4-(4-chlorophenoxy)-2-(trifluoromethyl)phenyl]-1-(1H-1,2,4-triazol-1-yl)butan-2-ol, (15.185) 2-[4-(4-chlorophenoxy)-2-(trifluoromethyl)phenyl]-1-(1H-1,2,4-triazol-1-yl)pentan-2-ol, (15.186) 2-[2-chloro-4-(4-chlorophenoxy)phenyl]-1-(1H-1,2,4-triazol-1-yl)butan-2-ol, (15.187) 2-[2-chloro-4-(2,4-dichlorophenoxy)phenyl]-1-(1H-1,2,4-triazol-1-yl)propan-2-ol, (15.188) 9-fluoro-2,2-dimethyl-5-(quinolin-3-yl)-2,3-dihydro-1,4-benzoxazepine, (15.189) 2-{2-fluoro-6-[(8-fluoro-2-methylquinolin-3-yl)oxy]phenyl}propan-2-ol, (15.190) 2-{2-[(7,8-difluoro-2-methylquinolin-3-yl)oxy]-6-fluorophenyl}propan-2-ol.

All named mixing partners of the classes (1) to (15) can, if their functional groups enable this, optionally form salts with suitable bases or acids.

As is known, some of the various kinds of advantages for plants, which have been mentioned above, can be combined, and generally applicable terms can be used to describe them. Such terms are, for example, the following names: phytotonic effect, resistance to stress factors, less plant stress, plant health, healthy plants, plant fitness, plant wellness, plant concept, vigor effect, stress shield, protective shield, crop health, crop health properties, crop health

products, crop health management, crop health therapy, plant health, plant health properties, plant health products, plant health management, plant health therapy, greening effect or regreening effect, freshness, or other terms with which a person skilled in the art is quite familiar.

In the context of the present invention, a good effect on resistance to abiotic stress is understood to mean, without limitation,

- at least an emergence improved by generally 3%, especially more than 5%, preferably more than 10%,
- at least a yield enhanced by generally 3%, especially more than 5%, preferably more than 10%,
- at least a root development improved by generally 3%, especially more than 5%, preferably more than 10%,
- at least a shoot size rising by generally 3%, especially more than 5%, preferably more than 10%,
- at least a leaf area increased by generally 3%, especially more than 5%, preferably more than 10%,
- at least an emergence improved by generally 3%, especially more than 5%, preferably more than 10%,
- at least a photosynthesis performance improved by generally 3%, especially more than 5%, preferably more than 10%, and/or
- at least a flower formation improved by generally 3%, especially more than 5%, preferably more than 10%,

and the effects may occur individually or else in any combination of two or more effects.

The present invention further provides a spray solution for treatment of plants, comprising an amount, effective for enhancing the resistance of plants to abiotic stress factors, preferably to drought stress, of at least one compound of formula (I) as herein defined or salts thereof. The spray solution may comprise other customary constituents, such as solvents, formulation aids, especially water. Further constituents may include active agrochemical ingredients described in detail below.

The present invention further provides for the use of corresponding spray solutions for increasing the resistance of plants to abiotic stress factors, preferably to drought stress. The remarks which follow apply both to the inventive use of at least one compound of formula (I) as herein defined or salts thereof, per se and to the corresponding spray solutions, and to the method of the invention comprising applying said compound, salt thereof, and corresponding spray solutions .

In accordance with the invention, it has additionally been found that the application, to plants or in their environment, of at least one compound of formula (I) as herein defined or salts thereof, in combination with at least one fertilizer as defined below is possible.

Fertilizers which can be used in accordance with the invention together with at least one compound of formula (I) as herein defined or salts thereof, are generally organic and inorganic nitrogen-containing compounds, for example ureas, urea/formaldehyde condensation products, amino acids, ammonium salts and ammonium nitrates, potassium salts (preferably chlorides, sulfates, nitrates), salts of phosphoric acid and/or salts of phosphorous acid (preferably potassium salts and ammonium salts). In this context, particular mention should be made of the NPK fertilizers, i.e. fertilizers which contain nitrogen, phosphorus and potassium, calcium ammonium nitrate, i.e. fertilizers which additionally contain calcium, or ammonium nitrate sulfate (formula $(NH_4)_2SO4$ $NH_4NO_3$), ammonium phosphate and ammonium sulfate. These fertilizers are common knowledge to those skilled in the art; see also, for example, Ullmann's Encyclopedia of Industrial Chemistry, 5th edition, Vol. A 10, pages 323 to 431, Verlagsgesellschaft, Weinheim, 1987.

The fertilizers may also contain salts of micronutrients (preferably calcium, sulfur, boron, manganese, magnesium, iron, boron, copper, zinc, molybdenum and cobalt) and phytohormones (for example vitamin B1 and indole-3-acetic acid) or mixtures thereof. Fertilizers used in accordance with the invention may also contain other salts such as monoammonium phosphate (MAP), diammonium phosphate (DAP), potassium sulfate, potassium chloride, magnesium sulfate. Suitable amounts of the secondary nutrients, or trace elements, are amounts of 0.5 to 5% by weight, based on the overall fertilizer. Further possible ingredients are crop protection compositions, insecticides or fungicides, growth regulators or mixtures thereof. This will be explained in more detail below.

The fertilizers can be used, for example, in the form of powders, granules, prills or compactates. However, the fertilizers can also be used in liquid form, dissolved in an aqueous medium. In this case, dilute aqueous ammonia can also be used as a nitrogen fertilizer. Further possible ingredients for fertilizers are described, for example, in Ullmann's Encyclopedia of Industrial Chemistry, 5th edition, 1987, volume A 10, pages 363 to 401, DE-A 41 28 828, DE-A 19 05 834 and DE-A 196 31 764. The general composition of the fertilizers which, within the context of the present invention, may take the form of straight and/or compound fertilizers, for example composed of nitrogen, potassium or phosphorus, may vary within a wide range. In general, a content of 1 to 30% by weight of nitrogen (preferably 5 to 20% by weight), 1 to 20% by weight of potassium (preferably 3 to 15% by weight) and a content of 1 to 20% by weight of phosphorus (preferably 3 to 10% by weight) is advantageous. The microelement content is usually in the ppm range, preferably in the range from 1 to 1000 ppm. In the context of the present invention, the fertilizer and the compound of formula (I) as herein defined or salt thereof may be administered simultaneously, i.e. synchronously. However, it is also possible first to apply the fertilizer and then the compound of formula (I) as herein defined or salt thereof, or first to apply the compound of formula (I) as herein defined or salt thereof and then the fertilizer. In the case of nonsynchronous application of

compound of formula (I) as herein defined or salt thereof and the fertilizer, the application in the context of the present invention is, however, effected in a functional relationship, especially within a period of generally 24 hours, preferably 18 hours, more preferably 12 hours, specifically 6 hours, more specifically 4 hours, even more specifically within 2 hours. In very particular embodiments of the present invention, compound of formula (I) as herein defined or salt thereof and the fertilizer are applied within a time frame of less than 1 hour, preferably less than 30 minutes, more preferably less than 15 minutes.

The compound of formula (I) as herein defined or salt thereof to be used in accordance with the invention, can preferably be applied to the following plants, if appropriate in combination with fertilizers, though the enumeration which follows is not limiting.

Preferred plants are those from the group of the useful plants, ornamental plants, turfgrass types, commonly used trees which are employed as ornamentals in public and domestic areas, and forestry trees. Forestry trees include trees for the production of timber, cellulose, paper and products made from parts of the trees. The term "useful plants" as used here refers to crop plants which are employed as plants for obtaining foods, animal feeds, fuels or for industrial purposes.

The useful plants include, for example, the following types of plants: triticale, durum (hard wheat), turf, vines, cereals, for example wheat, barley, rye, oats, hops, rice, corn and millet/sorghum; beet, for example sugar beet and fodder beet; fruits, for example pome fruit, stone fruit and soft fruit, for example apples, pears, plums, peaches, almonds, cherries and berries, for example strawberries, raspberries, blackberries; legumes, for example beans, lentils, peas and soybeans; oil crops, for example oilseed rape, mustard, poppies, olives, sunflowers, coconuts, castor oil plants, cacao beans and peanuts; cucurbits, for example pumpkin/squash, cucumbers and melons; fiber plants, for example cotton, flax, hemp and jute; citrus fruit, for example, oranges, lemons, grapefruit and tangerines; vegetables, for example spinach, lettuce, asparagus, cabbage species, carrots, onions, tomatoes, potatoes and bell peppers; Lauraceae, for example avocado, Cinnamonum, camphor, or also plants such as tobacco, nuts, coffee, aubergine, sugarcane, tea, pepper, grapevines, hops, bananas, latex plants and ornamentals, for example flowers, shrubs, deciduous trees and coniferous trees. This enumeration is not a limitation.

Particularly suitable target crops for the employment of the method and use according to the invention, i.e. the increase in stress tolerance by application of 4-phenybutyric acid and/or of one or more of the salts thereof, are considered to be the following plants: oats, rye, triticale, durum, cotton, aubergine, turf, pome fruit, stone fruit, soft fruit, corn, wheat, barley, cucumber, tobacco, vines, rice, cereals, pear, peppers, beans, soybeans, oilseed rape, tomato, bell pepper, melons, cabbage, potatoes and apples.

Examples of trees which can be improved in accordance with the method according to the invention include: Abies sp., Eucalyptus sp., Picea sp., Pinus sp., Aesculus sp., Platanus sp., Tilia sp., Acer sp., Tsuga sp., Fraxinus sp., Sorbus sp., Betula sp., Crataegus sp., Ulmus sp., Quercus sp., Fagus sp., Salix sp., Populus sp..

Preferred trees which can be improved by the method according to the invention include: from the tree species Aesculus: A. hippocastanum, A. pariflora, A. carnea; from the tree species Platanus: P. aceriflora, P. occidentalis, P. racemosa; from the tree species Picea: P. abies; from the tree species Pinus: P. radiate, P. ponderosa, P. contorta, P. sylvestre, P. elliottii, P. montecola, P. albicaulis, P. resinosa, P. palustris, P. taeda, P. flexilis, P. jeffregi, P. baksiana, P. strobes; from the tree species Eucalyptus: E. grandis, E. globulus, E. camadentis, E. nitens, E. obliqua, E. regnans, E. pilularus.

Particularly preferred trees which can be improved by the method according to the invention include: from the tree species Pinus: P. radiate, P. ponderosa, P. contorta, P. sylvestre, P. strobes; from the tree species Eucalyptus: E. grandis, E. globulus and E. camadentis.

Particularly preferred trees which can be improved by the method according to the invention include: horse chestnut, Platanaceae, linden tree, maple tree.

The present invention can also be applied to any turfgrass types, including cool-season turfgrasses and warm-season turfgrasses. Examples of cold-season turfgrasses are bluegrasses (Poa spp.), such as Kentucky bluegrass (Poa pratensis L.), rough bluegrass (Poa trivialis L.), Canada bluegrass (Poa compressa L.), annual bluegrass (Poa annua L.), upland bluegrass (Poa glaucantha Gaudin), wood bluegrass (Poa nemoralis L.) and bulbous bluegrass (Poa bulbosa L.); bentgrasses (Agrostis spp.) such as creeping bentgrass (Agrostis palustris Huds.), colonial bentgrass (Agrostis tenuis Sibth.), velvet bentgrass (Agrostis canina L.), South German Mixed Bentgrass (Agrostis spp. including Agrostis tenius Sibth., Agrostis canina L., and Agrostis palustris Huds.), and redtop (Agrostis alba L.);

fescues (Festuca spp.), such as red fescue (Festuca rubra L. spp. rubra), creeping fescue (Festuca rubra L.), chewings fescue (Festuca rubra commutata Gaud.), sheep fescue (Festuca ovina L.), hard fescue (Festuca longifolia Thuill.), hair fescue (Festucu capillata Lam.), tall fescue (Festuca arundinacea Schreb.) and meadow fescue (Festuca elanor L.);

ryegrasses (Lolium spp.), such as annual ryegrass (Lolium multiflorum Lam.), perennial ryegrass (Lolium perenne

L.) and italian ryegrass (Lolium multiflorum Lam.);

and wheatgrasses (Agropyron spp.), such as fairway wheatgrass (Agropyron cristatum (L.) Gaertn.), crested wheatgrass (Agropyron desertorum (Fisch.) Schult.) and western wheatgrass (Agropyron smithii Rydb.).

Examples of further cool-season turfgrasses are beachgrass (Ammophila breviligulata Fern.), smooth bromegrass (Bromus inermis Leyss.), cattails such as Timothy (Phleum pratense L.), sand cattail (Phleum subulatum L.), orchard grass (Dactylis glomerata L.), weeping alkaligrass (Puccinellia distans (L.) Parl.) and crested dog's-tail (Cynosurus cristatus L.).

Examples of warm-season turfgrasses are Bermuda grass (Cynodon spp. L. C. Rich), zoysia grass (Zoysia spp. Willd.), St. Augustine grass (Stenotaphrum secundatum Walt Kuntze), centipede grass (Eremochloa ophiuroides Munro Hack.), carpet grass (Axonopus affinis Chase), Bahia grass (Paspalum notatum Flugge), Kikuyu grass (Pennisetum clandestinum Hochst. ex Chiov.), buffalo grass (Buchloe dactyloids (Nutt.) Engelm.), Blue gramma (Bouteloua gracilis (H.B.K.) Lag. ex Griffiths), seashore paspalum (Paspalum vaginatum Swartz) and sideoats grama (Bouteloua curtipendula (Michx.) Torr.). Cool-season turfgrasses are generally preferred for the use in accordance with the invention. Particular preference is given to bluegrass, bentgrass and redtop, fescues and ryegrasses. Bentgrass is especially preferred.

In a particular embodiment, the present invention is applied to a plant selected from the group consisting of cotton, vine, cereals (such as wheat, rice, barley, triticale), corn, soybean, oilseed rape, sunflower, turf, horticultural crops, shrubs, fruit-trees and fruit-plants (such as apple-tree, peer-tree, citrus, banana, coffea, strawberry plant, raspberry plant), vegetables, particularly cereals, corn, oilseed rape, shrubs, fruit-trees and fruit-plants, vegetables and vines.

More preferably, plants of the plant cultivars which are commercially available or are in use are treated in accordance with the invention. Plant cultivars are to be understood as meaning plants having new properties ("traits") and which have been obtained by conventional breeding, by mutagenesis or with the aid of recombinant DNA techniques. Crop plants may accordingly be plants which can be obtained by conventional breeding and optimization methods or by biotechnological and genetic engineering methods or combinations of these methods, including the transgenic plants and including the plant varieties which are protectable and non-protectable by plant breeders' rights.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted microorganisms. This may, if appropriate, be one of the reasons of the enhanced activity of the combinations according to the invention, for example against fungi. Plantstrengthening (resistance-inducing) substances are to be understood as meaning, in the present context, those substances or combinations of substances which are capable of stimulating the defense system of plants in such a way that, when subsequently inoculated with unwanted microorganisms, the treated plants display a substantial degree of resistance to these microorganisms. In the present case, unwanted microorganisms are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding

and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Examples of nematode resistant plants are described in e.g. US Patent Application Nos 11/765,491, 11/765,494, 10/926,819, 10/782,020, 12/032,479, 10/783,417, 10/782,096, 11/657,964, 12/192,904, 11/396,808, 12/166,253, 12/166,239, 12/166,124, 12/166,209, 11/762,886, 12/364,335, 11/763,947, 12/252,453, 12/209,354, 12/491,396, 12/497,221, 12/644,632, 12/646,004, 12/701,058, 12/718,059, 12/721,595, 12/638,591 and in WO11/002992, WO11/014749, WO11/103247, WO11/103248. Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses). Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (WO 92/05251, WO 95/09910, WO 98/27806, WO 05/002324, WO 06/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 89/10396 in which, for example, a ribonuclease such as barnase is selectively expressed in the tapetum cells in the stamens. Fertility can then be restored by expression in the tapetum cells of a ribonuclease inhibitor such as barstar (e.g. WO 91/02069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium Salmonella typhimurium (Comai et al., 1983, Science 221, 370-371), the CP4 gene of the bacterium Agrobacterium sp. (Barry et al., 1992, Curr. Topics Plant Physiol. 7, 139-145), the genes encoding a Petunia EPSPS (Shah et al., 1986, Science 233, 478-481), a Tomato EPSPS (Gasser et al., 1988, J. Biol. Chem. 263, 4280-4289), or an Eleusine EPSPS (WO 01/66704). It can also be a mutated EPSPS as described in for example

EP 0837944, WO 00/66746, WO 00/66747, WO02/26995, WO11/000498. Glyphosate-tolerant plants can also be obtained by expressing a gene that encodes a glyphosate oxido-reductase enzyme as described in U.S. Patent Nos. 5,776,760 and 5,463,175. Glyphosate-tolerant plants can also be obtained by expressing a gene that encodes a glyphosate acetyl transferase enzyme as described in for example WO 02/36782, WO 03/092360, WO 05/012515 and WO 07/024782. Glyphosate-tolerant plants can also be obtained by selecting plants containing naturally-occurring mutations of the abovementioned genes, as described in for example WO 01/024615 or WO 03/013226. Plants expressing EPSPS

genes that confer glyphosate tolerance are described in e.g. US Patent Application Nos 11/517,991, 10/739,610, 12/139,408, 12/352,532, 11/312,866, 11/315,678, 12/421,292, 11/400,598, 11/651,752, 11/681,285, 11/605,824, 12/468,205, 11/760,570, 11/762,526, 11/769,327, 11/769,255, 11/943801 or 12/362,774. Plants comprising other genes that confer glyphosate tolerance, such as decarboxylase genes, are described in e.g. US patent applications 11/588,811, 11/185,342, 12/364,724, 11/185,560 or 12/423,926.

Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition, e.g. described in US Patent Application No 11/760,602. One such efficient detoxifying enzyme is an enzyme encoding a phosphinothricin acetyltransferase (such as the bar or pat protein from Streptomyces species). Plants expressing an exogenous phosphinothricin acetyltransferase are for example described in U.S. Patent Nos. 5,561,236; 5,648,477; 5,646,024; 5,273,894; 5,637,489; 5,276,268; 5,739,082; 5,908,810 and 7,112,665. Further herbicide-tolerant plants are also plants that are made tolerant to the herbicides inhibiting the enzyme hydroxyphenylpyruvatedioxygenase (HPPD). HPPD is an enzyme that catalyze the reaction in which para-hydroxyphenylpyruvate (HPP) is transformed into homogentisate. Plants tolerant to HPPD-inhibitors can be transformed with a gene encoding a naturally-occurring resistant HPPD enzyme, or a gene encoding a mutated or chimeric HPPD enzyme as described in WO 96/38567, WO 99/24585, WO 99/24586, WO 2009/144079, WO 2002/046387, or US 6,768,044, WO11/076877, WO11/076882, WO11/076885, WO11/076889, WO11/076892. Tolerance to HPPD-inhibitors can also be obtained by transforming plants with genes encoding certain enzymes enabling the formation of homogentisate despite the inhibition of the native HPPD enzyme by the HPPD-inhibitor. Such plants and genes are described in WO 99/34008 and WO 02/36787. Tolerance of plants to HPPD inhibitors can also be improved by transforming plants with a gene encoding an enzyme having prephenate deshydrogenase (PDH) activity in addition to a gene encoding an HPPD-tolerant enzyme, as described in WO 2004/024928. Further, plants can be made more tolerant to HPPD-inhibitor herbicides by adding into their genome a gene encoding an enzyme capable of metabolizing or degrading HPPD inhibitors, such as the CYP450 enzymes shown in WO 2007/103567 and WO 2008/150473.

Still further herbicide resistant plants are plants that are made tolerant to acetolactate synthase (ALS) inhibitors. Known ALS-inhibitors include, for example, sulfonylurea, imidazolinone, triazolopyrimidines, pryimidinyoxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright (2002, Weed Science 50:700-712), but also, in U.S. Patent No. 5,605,011, 5,378,824, 5,141,870, and 5,013,659. The production of sulfonylurea-tolerant plants and imidazolinone-tolerant plants is described in U.S. Patent Nos. 5,605,011; 5,013,659; 5,141,870; 5,767,361; 5,731,180; 5,304,732; 4,761,373; 5,331,107; 5,928,937; and 5,378,824; and international publication WO 96/33270. Other imidazolinone-tolerant plants are also described in for example

WO 2004/040012, WO 2004/106529, WO 2005/020673, WO 2005/093093, WO 2006/007373,
WO 2006/015376, WO 2006/024351, and WO 2006/060634. Further sulfonylurea- and imidazolinone-tolerant plants are also described in for example WO 07/024782, WO11/076345,
WO2012058223 and US Patent Application No 61/288958.

Other plants tolerant to imidazolinone and/or sulfonylurea can be obtained by induced mutagenesis, selection in cell cultures in the presence of the herbicide or mutation breeding as described for example for soybeans in U.S. Patent 5,084,082, for rice in WO 97/41218, for sugar beet in U.S. Patent 5,773,702 and WO 99/057965, for lettuce in U.S. Patent 5,198,599, or for sunflower in WO 01/065922.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

An "insect-resistant transgenic plant", as used herein, includes any plant containing at least one transgene comprising a coding sequence encoding:

1) an insecticidal crystal protein from Bacillus thuringiensis or an insecticidal portion thereof, such as the insecticidal crystal proteins listed by Crickmore et al. (1998, Microbiology and Molecular Biology Reviews, 62: 807-813), updated by Crickmore et al. (2005) at the Bacillus thuringiensis toxin nomenclature, online at: http://www.lifesci.sussex.ac.uk/Home/Neil_Crickmore/Bt/), or insecticidal portions thereof, e.g., proteins of the Cry protein classes Cry1Ab, Cry1Ac, Cry1B, Cry1C, Cry1D, Cry1F, Cry2Ab, Cry3Aa, or Cry3Bb or insecticidal portions thereof (e.g. EP 1999141 and WO 2007/107302), or such proteins encoded by synthetic genes as e.g. described in and US Patent Application No 12/249,016 ; or

2) a crystal protein from Bacillus thuringiensis or a portion thereof which is insecticidal in the presence of a second other crystal protein from Bacillus thuringiensis or a portion thereof, such as the binary toxin made up of the Cry34 and Cry35 crystal proteins (Moellenbeck et al. 2001, Nat. Biotechnol. 19: 668-72; Schnepf et al. 2006, Applied Environm. Microbiol. 71, 1765-1774) or the binary toxin made up of the Cry1A or CrylF proteins and the Cry2Aa or Cry2Ab or Cry2Ae proteins (US Patent Appl. No. 12/214,022 and EP 08010791.5); or

3) a hybrid insecticidal protein comprising parts of different insecticidal crystal proteins from Bacillus thuringiensis, such as a hybrid of the proteins of 1) above or a hybrid of the proteins of 2) above, e.g., the Cry1A.105 protein produced by corn event MON89034 (WO 2007/027777); or

4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604; or

5) an insecticidal secreted protein from Bacillus thuringiensis or Bacillus cereus, or an insecticidal portion thereof, such as the vegetative insecticidal (VIP) proteins listed at: http://www.lifesci.sussex.ac.uk/home/Neil_Crickmore/Bt/vip.html, e.g., proteins from the VIP3Aa protein class; or

6) a secreted protein from Bacillus thuringiensis or Bacillus cereus which is insecticidal in the presence of a second secreted protein from Bacillus thuringiensis or B. cereus, such as the binary toxin made up of the VIP1A and VIP2A proteins (WO 94/21795); or

7) a hybrid insecticidal protein comprising parts from different secreted proteins from Bacillus thuringiensis or Bacillus cereus, such as a hybrid of the proteins in 1) above or a hybrid of the proteins in 2) above; or

8) a protein of any one of 5) to 7) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102; or

9) a secreted protein from Bacillus thuringiensis or Bacillus cereus which is insecticidal in the presence of a crystal protein from Bacillus thuringiensis, such as the binary toxin made up of VIP3 and Cry1A or Cry1F (US Patent Appl. No. 61/126083 and 61/195019), or the binary toxin made up of the VIP3 protein and the Cry2Aa or Cry2Ab or Cry2Ae proteins (US Patent Appl. No. 12/214,022 and EP 08010791.5).

10) a protein of 9) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein)

Of course, an insect-resistant transgenic plant, as used herein, also includes any plant comprising a combination of genes encoding the proteins of any one of the above classes 1 to 10. In one embodiment, an insect-resistant plant contains more than one transgene encoding a protein of any one of the above classes 1 to 10, to expand the range of target insect species affected when using different proteins directed at different target insect species, or to delay insect resistance development to the plants by using different proteins insecticidal to the same target insect species but having a different mode of action, such as binding to different receptor binding sites in the insect.

An "insect-resistant transgenic plant", as used herein, further includes any plant containing at least one transgene comprising a sequence producing upon expression a double-stranded RNA which upon ingestion by a plant insect pest inhibits the growth of this insect pest, as described e.g. in WO 2007/080126, WO 2006/129204, WO 2007/074405, WO 2007/080127 and WO 2007/035650.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:

1) plants which contain a transgene capable of reducing the expression and/or the activity of poly(ADP-ribose) polymerase (PARP) gene in the plant cells or plants as described in WO 00/04173, WO/2006/045633, EP 04077984.5, or EP 06009836.5.

2) plants which contain a stress tolerance enhancing transgene capable of reducing the expression and/or the activity of the PARG encoding genes of the plants or plants cells, as described e.g. in WO 2004/090140.

3) plants which contain a stress tolerance enhancing transgene coding for a plant-functional enzyme of the nicotineamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphorybosyltransferase as described e.g. in EP 04077624.7, WO 2006/133827,

PCT/EP07/002433, EP 1999263, or WO 2007/107326.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :

1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP 0571427, WO 95/04826, EP 0719338, WO 96/15248, WO 96/19581, WO 96/27674, WO 97/11188, WO 97/26362, WO 97/32985, WO 97/42328, WO 97/44472, WO 97/45545, WO 98/27212, WO 98/40503, WO99/58688, WO 99/58690, WO 99/58654, WO 00/08184, WO 00/08185, WO 00/08175, WO 00/28052, WO 00/77229, WO 01/12782, WO 01/12826, WO 02/101059, WO 03/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 00/22140, WO 2006/063862, WO 2006/072603, WO 02/034923, EP 06090134.5, EP 06090228.5, EP 06090227.7, EP 07090007.1, EP 07090009.7, WO 01/14569, WO 02/79410, WO 03/33540, WO 2004/078983, WO 01/19975, WO 95/26407, WO 96/34968, WO 98/20145, WO 99/12950, WO 99/66050, WO 99/53072, US 6,734,341, WO 00/11192, WO 98/22604, WO 98/32326, WO 01/98509, WO 01/98509, WO 2005/002359, US 5,824,790, US 6,013,861, WO 94/04693, WO 94/09144, WO 94/11520, WO 95/35026, WO 97/20936, WO 10/012796, WO 10/003701

2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP 0663956, WO 96/01904, WO 96/21023, WO 98/39460, and WO 99/24593, plants producing alpha-1,4-glucans as disclosed in WO 95/31553, US 2002031826, US 6,284,479, US 5,712,107, WO 97/47806, WO 97/47807, WO 97/47808 and WO 00/14249, plants producing alpha-1,6 branched alpha-1,4-glucans, as disclosed in WO 00/73422, plants producing alternan, as disclosed in e.g. WO 00/47727, WO 00/73422, EP 06077301.7, US 5,908,975 and EP 0728213,

3) transgenic plants which produce hyaluronan, as for example disclosed in WO 2006/032538, WO 2007/039314, WO 2007/039315, WO 2007/039316, JP 2006304779, and WO 2005/012529.

4) transgenic plants or hybrid plants, such as onions with characteristics such as 'high soluble solids content', 'low pungency' (LP) and/or 'long storage' (LS), as described in US Patent Appl. No. 12/020,360 and 61/054,026.

5) Transgenic plants displaying an increase yield as for example disclosed in WO11/095528

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:

a) Plants, such as cotton plants, containing an altered form of cellulose synthase genes as described in WO 98/00549

b) Plants, such as cotton plants, containing an altered form of rsw2 or rsw3 homologous nucleic acids as described in WO 2004/053219

c) Plants, such as cotton plants, with increased expression of sucrose phosphate synthase as described in WO 01/17333

d) Plants, such as cotton plants, with increased expression of sucrose synthase as described in WO 02/45485

e) Plants, such as cotton plants, wherein the timing of the plasmodesmatal gating at the basis of the fiber cell is altered, e.g. through downregulation of fiber-selective β-1,3-glucanase as described in WO 2005/017157, or as described in EP 08075514.3 or US Patent Appl. No. 61/128,938

f) Plants, such as cotton plants, having fibers with altered reactivity, e.g. through the expression of N-acetylglucosaminetransferase gene including nodC and chitin synthase genes as described in WO 2006/136351 WO11/089021, WO2012074868

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics and include:

a) Plants, such as oilseed rape plants, producing oil having a high oleic acid content as described e.g. in US 5,969,169, US 5,840,946 or US 6,323,392 or US 6,063,947

b) Plants such as oilseed rape plants, producing oil having a low linolenic acid content as described in US 6,270,828, US 6,169,190, US 5,965,755, or WO11/060946.

c) Plant such as oilseed rape plants, producing oil having a low level of saturated fatty acids as described e.g. in US Patent No. 5,434,283 or US Patent Application No 12/668303

d) Plants such as oilseed rape plants, producing oil having an aleter glucosinolate content as described in WO2012075426.

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering as described in US Patent Appl. No. 61/135,230, WO09/068313, WO10/006732 and WO2012090499.

Plants or plant cultivars (that can be obtained by plant biotechnology methods such as genetic engineering) which may also be treated according to the invention are plants, such as Tobacco plants, with altered post-translational protein modification patterns, for example as described in WO 10/121818 and WO 10/145846

Particularly useful transgenic plants which may be treated according to the invention are plants containing transformation events, or combination of transformation events, that are the subject of petitions for non-regulated status, in the United States of America, to the Animal and Plant Health Inspection Service (APHIS) of the United States Department of Agriculture (USDA) whether such petitions are granted or are still pending. At any time this information is readily available from APHIS (4700 River Road Riverdale, MD 20737, USA), for instance on its internet site (URL http://www.aphis.usda.gov/brs/not_reg.html). On the filing date of this application the petitions for nonregulated status that were pending with APHIS or granted by APHIS were those which contains the following information:

- Petition : the identification number of the petition. Technical descriptions of the transformation events can be found in the individual petition documents which are obtainable from APHIS, for example on the APHIS website, by reference to this petition number. These descriptions are herein incorporated by reference.
- Extension of Petition : reference to a previous petition for which an extension is requested.
- Institution : the name of the entity submitting the petition.
- Regulated article : the plant species concerned.
- Transgenic phenotype : the trait conferred to the plants by the transformation event.
- Transformation event or line : the name of the event or events (sometimes also designated as lines or lines) for which nonregulated status is requested.
- APHIS documents : various documents published by APHIS in relation to the Petition and which can be requested with APHIS.

Additional particularly useful plants containing single transformation events or combinations of transformation events are listed for example in the databases from various national or regional regulatory agencies (see for example http://gmoinfo.irc.it/gmp browse.aspx and http://www.agbios.com/dbase .php).

Particularly useful transgenic plants which may be treated according to the invention are plants containing transformation events, or a combination of transformation events, and that are listed for example in the databases for various national or regional regulatory agencies including Event 1143-14A (cotton, insect control, not deposited, described in WO2006/128569); Event 1143-51B (cotton, insect control, not deposited, described in WO2006/128570); Event 1445 (cotton, herbicide tolerance, not deposited, described in US2002120964 or WO2002/034946); Event 17053 (rice, herbicide tolerance, deposited as PTA-9843, described in WO2010/117737); Event 17314 (rice, herbicide tolerance, deposited as PTA-9844, described in WO2010/117735); Event 281-24-236 (cotton, insect control - herbicide tolerance, deposited as PTA-6233, described in WO2005/103266 or US2005216969); Event 3006-210-23 (cotton, insect control - herbicide tolerance, deposited as PTA-6233, described in US2007143876 or WO2005/103266); Event 3272 (corn, quality trait, deposited as PTA-9972, described in WO2006098952 or US2006230473); Event 40416 (corn, insect control - herbicide tolerance, deposited as ATCC PTA-11508, described in WO2011/075593); Event 43A47 (corn, insect control - herbicide tolerance, deposited as ATCC PTA-11509, described in WO2011/075595); Event 5307 (corn, insect control, deposited as ATCC PTA-9561, described in WO2010/077816); Event ASR-368 (bent grass, herbicide tolerance, deposited as ATCC PTA-4816, described in US2006162007 or WO2004053062); Event B16 (corn, herbicide tolerance, not deposited, described in US2003126634); Event BPS-CV127-9 (soybean, herbicide tolerance, deposited as NCIMB No. 41603, described in WO2010/080829); Event CE43-67B (cotton, insect control, deposited as DSM ACC2724, described in US2009217423 or WO2006/128573); Event CE44-69D (cotton, insect control, not deposited, described in

US20100024077); Event CE44-69D (cotton, insect control, not deposited, described in WO2006/128571); Event CE46-02A (cotton, insect control, not deposited, described in WO2006/128572); Event COT102 (cotton, insect control, not deposited, described in US2006130175 or WO2004039986); Event COT202 (cotton, insect control, not deposited, described in US2007067868 or WO2005054479); Event COT203 (cotton, insect control, not deposited, described in WO2005/054480); Event DAS40278 (corn, herbicide tolerance, deposited as ATCC PTA-10244, described in WO2011/022469); Event DAS-59122-7 (corn, insect control - herbicide tolerance, deposited as ATCC PTA 11384 , described in US2006070139); Event DAS-59132 (corn, insect control - herbicide tolerance, not deposited, described in WO2009/100188); Event DAS68416 (soybean, herbicide tolerance, deposited as ATCC PTA-10442, described in WO2011/066384 or WO2011/066360); Event DP-098140-6 (corn, herbicide tolerance, deposited as ATCC PTA-8296, described in US2009137395 or WO2008/112019); Event DP-305423-1 (soybean, quality trait, not deposited, described in US2008312082 or WO2008/054747); Event DP-32138-1 (corn, hybridization system, deposited as ATCC PTA-9158, described in US20090210970 or WO2009/103049); Event DP-356043-5 (soybean, herbicide tolerance, deposited as ATCC PTA-8287, described in US20100184079 or WO2008/002872); Event EE-1 (brinjal, insect control, not deposited, described in WO2007/091277); Event FI117 (corn, herbicide tolerance, deposited as ATCC 209031, described in US2006059581 or WO1998/044140); Event GA21 (corn, herbicide tolerance, deposited as ATCC 209033, described in US2005086719 or WO1998/044140); Event GG25 (corn, herbicide tolerance, deposited as ATCC 209032, described in US2005188434 or WO1998/044140); Event GHB119 (cotton, insect control - herbicide tolerance, deposited as ATCC PTA-8398, described in WO2008/151780); Event GHB614 (cotton, herbicide tolerance, deposited as ATCC PTA-6878, described in US2010050282 or WO2007/017186); Event GJ11 (corn, herbicide tolerance, deposited as ATCC 209030, described in US2005188434 or WO1998/044140); Event GM RZ13 (sugar beet, virus resistance , deposited as NCIMB-41601, described in WO2010/076212); Event H7-1 (sugar beet, herbicide tolerance, deposited as NCIMB 41158 or NCIMB 41159, described in US2004172669 or WO2004/074492); Event JOPLIN1 (wheat, disease tolerance, not deposited, described in US2008064032); Event LL27 (soybean, herbicide tolerance, deposited as NCIMB41658, described in WO2006/108674 or US2008320616); Event LL55 (soybean, herbicide tolerance, deposited as NCIMB 41660, described in WO2006/108675 or US2008196127); Event LLcotton25 (cotton, herbicide tolerance, deposited as ATCC PTA-3343, described in WO2003013224 or US2003097687); Event LLRICE06 (rice, herbicide tolerance, deposited as ATCC-23352, described in US6468747 or WO2000/026345); Event LLRICE601 (rice, herbicide tolerance, deposited as ATCC PTA-2600, described in US20082289060 or WO2000/026356); Event LY038 (corn, quality trait, deposited as ATCC PTA-5623, described in US2007028322 or WO2005061720); Event MIR162 (corn, insect control, deposited as PTA-8166, described in US2009300784 or WO2007/142840); Event MIR604 (corn, insect control, not deposited, described in US2008167456 or WO2005103301); Event MON15985 (cotton, insect control, deposited as ATCC PTA-2516, described in US2004-250317 or WO2002/100163); Event MON810 (corn, insect control, not deposited, described in US2002102582); Event MON863 (corn, insect control, deposited as ATCC PTA-2605, described in WO2004/011601 or US2006095986); Event MON87427 (corn, pollination control, deposited as ATCC PTA-7899, described in WO2011/062904); Event MON87460 (corn, stress tolerance, deposited as ATCC PTA-8910, described in WO2009/111263 or US20110138504); Event MON87701 (soybean, insect control, deposited as ATCC PTA-8194, described in US2009130071 or WO2009/064652); Event MON87705 (soybean, quality trait - herbicide tolerance, deposited as ATCC PTA-9241, described in US20100080887 or WO2010/037016); Event MON87708 (soybean, herbicide tolerance, deposited as ATCC PTA9670, described in WO2011/034704); Event MON87754 (soybean, quality trait, deposited as ATCC PTA-9385, described in WO2010/024976); Event MON87769 (soybean, quality trait, deposited as ATCC PTA-8911, described in US20110067141 or WO2009/102873); Event MON88017 (corn, insect control - herbicide tolerance, deposited as ATCC PTA-5582, described in US2008028482 or WO2005/059103); Event MON88913 (cotton, herbicide tolerance, deposited as ATCC PTA-4854, described in WO2004/072235 or US2006059590); Event MON89034 (corn, insect control, deposited as ATCC PTA-7455, described in WO2007/140256 or US2008260932); Event MON89788 (soybean, herbicide tolerance, deposited as ATCC PTA-6708, described in US2006282915 or WO2006/130436); Event MS11 (oilseed rape, pollination control - herbicide tolerance, deposited as ATCC PTA-850 or PTA-2485, described in WO2001/031042); Event MS8, (oilseed rape, pollination control - herbicide tolerance, deposited as ATCC PTA-730, described in WO2001/041558 or US2003188347); Event NK603 (corn, herbicide tolerance, deposited as ATCC PTA-2478, described in US2007-292854); Event PE-7 (rice, insect control, not deposited, described in WO2008/114282); Event RF3, (oilseed rape, pollination control - herbicide tolerance, deposited as ATCC PTA-730, described in WO2001/041558 or US2003188347); Event RT73 (oilseed rape, herbicide tolerance, not deposited, described in WO2002/036831 or US2008070260); Event T227-1 (sugar beet, herbicide tolerance, not deposited, described in WO2002/44407 or US2009265817); Event T25 (corn, herbicide tolerance, not deposited, described in US2001029014 or WO2001/051654); Event T304-40 (cotton, insect control - herbicide tolerance, deposited as ATCC PTA-8171, described in US2010077501 or WO2008/122406); Event T342-142 (cotton, insect control, not deposited, described in WO2006/128568); Event TC1507 (corn, insect control - herbicide tolerance, not deposited, described in US2005039226 or WO2004/099447); Event VIP1034 (corn, insect control - herbicide tolerance, deposited as ATCC PTA-3925., described in WO2003/052073), Event 32316 (corn,insect control-herbicide tolerance,deposited as PTA-11507, described in

WO2011/153186A1), Event 4114 (corn, insect control-herbicide tolerance,deposited as PTA-11506, described in WO2011/084621), event EE-GM3 / FG72 (soybean, herbicide tolerance, ATCC Accession N° PTA-11041, WO2011/063413A2), event DAS-68416-4 (soybean, herbicide tolerance, ATCC Accession N° PTA-10442, WO2011/066360A1), event DAS-68416-4 (soybean, herbicide tolerance, ATCC Accession N° PTA-10442, WO2011/066384A1), event DP-040416-8 (corn, insect control, ATCC Accession N° PTA-11508, WO2011/075593A1), event DP-043A47-3 (corn, insect control, ATCC Accession N° PTA-11509, WO2011/075595A1), event DP-004114-3 (corn, insect control, ATCC Accession N° PTA-11506, WO2011/084621A1), event DP-032316-8 (corn, insect control, ATCC Accession N° PTA-11507, WO2011/084632A1), event MON-88302-9 (oilseed rape, herbicide tolerance, ATCC Accession N° PTA-10955, WO2011/153186A1), event DAS-21606-3 (soybean, herbicide tolerance, ATCC Accession No. PTA-11028, WO2012/033794A2), event MON-87712-4 (soybean, quality trait, ATCC Accession N°. PTA-10296, WO2012/051199A2), event DAS-44406-6 (soybean, stacked herbicide tolerance, ATCC Accession N°. PTA-11336, WO2012/075426A1), event DAS-14536-7 (soybean, stacked herbicide tolerance, ATCC Accession N°. PTA-11335, WO2012/075429A1), event SYN-000H2-5 (soybean, herbicide tolerance, ATCC Accession N°. PTA-11226, WO2012/082548A2), event DP-061061-7 (oilseed rape, herbicide tolerance, no deposit N° available, WO2012071039A1), event DP-073496-4 (oilseed rape, herbicide tolerance, no deposit N° available, US2012131692), event 8264.44.06.1 (soybean, stacked herbicide tolerance, Accession N° PTA-11336, WO2012075426A2), event 8291.45.36.2 (soybean, stacked herbicide tolerance, Accession N°. PTA-11335, WO2012075429A2).

The compound of the formula (I), or salt thereof, to be used in accordance with the invention can be converted to customary formulations, such as solutions, emulsions, wettable powders, water- and oilbased suspensions, powders, dusts, pastes, soluble powders, soluble granules, granules for broadcasting, suspoemulsion concentrates, natural compounds impregnated with active ingredient, synthetic substances impregnated with active ingredient, fertilizers, and also microencapsulations in polymeric substances. In the context of the present invention, it is especially preferred when 4-phenylbutyric acid or salts thereof, of the formula (I), are used in the form of a spray formulation.

The present invention therefore additionally also relates to a spray formulation for enhancing the resistance of plants to abiotic stress, preferably to drought stress. A spray formulation is described in detail hereinafter:

The formulations for spray application are produced in a known manner, for example by mixing the 4-phenylbutyric acid or salts thereof, of the formula (I) with extenders, i.e. liquid solvents and/or solid carriers, optionally with use of surfactants, i.e. emulsifiers and/or dispersants and/or foam formers. Further customary additives, for example customary extenders and solvents or diluents, dyes, wetting agents, dispersants, emulsifiers, antifoams, preservatives, secondary thickeners, stickers, gibberellins and also water, can optionally also be used. The formulations are produced either in suitable plants or else before or during application.

The auxiliaries used may be those substances which are suitable for imparting, to the composition itself and/or to preparations derived therefrom (for example spray liquors), particular properties such as particular technical properties and/or else special biological properties. Typical auxiliaries include: extenders, solvents and carriers.

Suitable extenders are, for example, water, polar and nonpolar organic chemical liquids, for example from the classes of the aromatic and nonaromatic hydrocarbons (such as paraffins, alkylbenzenes, alkylnaphthalenes, chlorobenzenes), the alcohols and polyols (which may optionally also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulfones and sulfoxides (such as dimethyl sulfoxide).

If the extender utilized is water, it is also possible to use, for example, organic solvents as auxiliary solvents. Useful liquid solvents essentially include: aromatics such as xylene, toluene or alkylnaphthalenes, chlorinated aromatics and chlorinated aliphatic hydrocarbons such as chlorobenzenes, chloroethylenes or methylene chloride, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethyl sulfoxide, and also water.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Useful wetting agents which may be present in the formulations usable in accordance with the invention are all substances which promote wetting and which are conventionally used for the formulation of active agrochemical ingredients. Preference is given to using alkyl naphthalenesulfonates, such as diisopropyl or diisobutyl naphthalenesulfonates.

Useful dispersants and/or emulsifiers which may be present in the formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Suitable nonionic dispersants are especially ethylene oxide/propylene oxide block polymers, alkylphenol polyglycol ethers and tristryrylphenol polyglycol ether, and the phosphated or sulfated derivatives thereof. Suitable anionic dispersants are especially lignosulfonates, polyacrylic acid salts and arylsulfonate/formaldehyde condensates.

Antifoams which may be present in the formulations usable in accordance with the invention are all foam-inhibiting substances conventionally used for the formulation of active agrochemical ingredients. Silicone antifoams and magnesium stearate can be used with preference.

Preservatives which may be present in the formulations usable in accordance with the invention are all substances usable for such purposes in agrochemical compositions. Examples include dichlorophene and benzyl alcohol hemiformal.

Secondary thickeners which may be present in the formulations usable in accordance with the invention are all substances usable for such purposes in agrochemical compositions. Preferred examples include cellulose derivatives, acrylic acid derivatives, xanthan, modified clays and finely divided silica.

Stickers which may be present in the formulations usable in accordance with the invention include all customary binders usable in seed-dressing products. Preferred examples include polyvinylpyrrolidone, polyvinyl acetate, polyvinyl alcohol and tylose. Gibberellins which may be present in the formulations usable in accordance with the invention may preferably be gibberellins A1, A3 (= gibberellic acid), A4 and A7; particular preference is given to using gibberellic acid. The gibberellins are known (cf. R. Wegler "Chemie der Pflanzenschutz- and Schädlingsbekämpfungsmittel" [Chemistry of the Crop Protection Compositions and Pesticides], vol. 2, Springer Verlag, 1970, p. 401-412).

Further additives may be fragrances, mineral or vegetable, optionally modified oils, waxes and nutrients (including trace nutrients), such as salts of iron, manganese, boron, copper, cobalt, molybdenum and zinc. Additionally present may be stabilizers, such as cold stabilizers, antioxidants, light stabilizers or other agents which improve chemical and/or physical stability.

The formulations contain generally between 0.01 and 98% by weight, preferably between 0.5 and 90 %, of the 4-phenylbutyric acid and/or salts thereof, of the formula (I).

The compound of formula (I) as herein defined or salt thereof can be present in commercially available formulations and also in the use forms, prepared from these formulations, as a mixture with other active compounds, such as insecticides, attractants, sterilizing agents, bactericides, acaricides, nematicides, fungicides, growth-regulating substances, herbicides, safeners, fertilizers or semiochemicals.

In addition, the described positive effect of compound of formula (I) as herein defined or salt thereof on the plants' own defenses can be supported by an additional treatment with active insecticidal, fungicidal or bactericidal ingredients.

Preferred times for the application of compound of formula (I) as herein defined or salt thereof for enhancing resistance to abiotic stress, preferably to drought stress, are treatments of the soil, stems and/or leaves with the approved application rates.

The compound of formula (I) as herein defined or salt thereof may generally additionally be present in their commercial formulations and in the use forms prepared from these formulations in mixtures with other active ingredients, such as insecticides, attractants, sterilants, acaricides, nematicides, fungicides, growth regulators, substances which influence plant maturity, safeners, herbicides or biologics.

The preparation and the use of the inventive compounds is illustrated by the examples which follow.

N-cyclopropyl amides of formula (I) wherein T represents an oxygen atom, can be prepared by condensation of a substituted N-cyclopropyl benzylamine with 3-(difluoromethyl)-5-fluoro-1-methyl-1H-pyrazole-4-carbonyl chloride according to WO-2007/087906 (process P1) and WO-2010/130767 (process PI - step 10).

Substituted N-cyclopropyl benzylamines are known or can be prepared by known processes such as the reductive amination of a substituted aldehyde with cyclopropanamine (J. Med. Chem., 2012, 55 (1), 169-196) or by nucleophilic substitution of a substituted benzyl alkyl (or aryl)sulfonate or a substituted benzyl halide with cyclopropanamine (Bioorg. Med. Chem., 2006, 14, 8506-8518 and WO-2009/140769).

3-(difluoromethyl)-5-fluoro-1-methyl-1H-pyrazole-4-carbonyl chloride can be prepared according to WO-2010/130767 (process PI - steps 9 or 11)

N-cyclopropyl thioamides of formula (I) wherein T represents a sulfur atom, can be prepared by thionation of a N-cyclopropyl amide of formula (I) wherein T represents a oxygen atom, according to WO-2009/016220 (process P1) and WO-2010/130767 (process P3).

The following examples illustrate in a non limiting manner the preparation of the compounds of formula (I) according to the invention.

<u>Preparation of N-cyclopropyl-3-(difluoromethyl)-5-fluoro-N-(2-isopropylbenzyl)-1-methyl-1H-pyrazole-4-carboxamide</u> (compound A1)

Step A : preparation of N-(2-isopropylbenzyl)cyclopropanamine

To a solution of 55.5 g (971 mmol) of cyclopropanamine in 900 mL of methanol, are successively added 20 g

of 3 Å molecular sieves and 73 g (1.21 mol) of acetic acid. 72 g (486 mmol) of 2-isopropyl-benzaldehyde are then added dropwise and the reaction mixture is further heated at reflux for 4 hours.

The reaction mixture is then cooled to 0 °C and 45.8 g (729 mmol) of sodium cyanoborohydride are added by portion in 10 min and the reaction mixture is stirred again for 3 hours at reflux. The cooled reaction mixture is filtered over a cake of diatomaceous earth. The cake is washed abundantly by methanol and the methanolic extracts are concentrated under vacuum. Water is then added to the residue and the pH is adjusted to 12 with 400 mL of a 1 N aqueous solution of sodium hydroxide. The watery layer is extracted with ethyl acetate, washed by water (2 x 300 mL) and dried over magnesium sulfate to yield 81.6 g (88%) of N-(2-isopropylbenzyl)cyclopropanamine as a yellow oil used as such in the next step.

The hydrochloride salt can be prepared by dissolving N-(2-isopropylbenzyl)cyclopropanamine in diethyl-ether (1.4 mL / g) at 0 °C followed by addition of a 2 M solution of hydrochloric acid in diethylether (1.05 eq.). After a 2 hours stirring, N-(2-isopropylbenzyl)cyclopropanamine hydrochloride (1:1) is filtered off, washed by diethylether and dried under vacuum at 40 °C for 48 hours. Mp (melting point) = 149 °C

Step B : preparation of N-cyclopropyl-3-(difluoromethyl)-5-fluoro-N-(2-isopropylbenzyl)-1-methyl-1H -pyrazole-4-carboxamide

To 40.8 g (192 mmol) of N-(2-isopropylbenzyl)cyclopropanamine in 1 L of dry tetrahydrofurane are added at room temperature, 51 mL (366 mmol) of triethylamine. A solution of 39.4 g (174 mmol) of 3-(difluoromethyl)-5-fluoro-1-methyl-1H-pyrazole-4-carbonyl chloride in 800 mL of dry tetrahydrofurane is then added dropwise while maintaining the temperature below 34 °C. The reaction mixture is heated at reflux for 2 hours then left overnight at room temperature. Salts are filtered off and the filtrate is concentrated under vacuum to yield 78.7 g of a brown oil. Column chromatography on silica gel (750 g - gradient n-heptane/ethyl acetate) yields 53 g (71% yield) of N-cyclopropyl-3-(difluoromethyl)-5-fluoro-N-(2-isopropylbenzyl)-1-methyl-1H-pyrazole-4-carboxamide as a yellow oil that slowly crystallizes. Mp = 76-79 °C.

In the same way, compounds A2 to A19 can be prepared according to the preparation described for compound A1.

Preparation of N-cyclopropyl-3-(difluoromethyl)-5-fluoro-N-(2-isopropylbenzyl)-1-methyl-1H-pyrazole-4-carbothioamide (compound A20)

A solution of 14.6 g (65 mmol) of phosphorus pentasulfide and 48 g (131 mmol) of N-cyclopropyl-3-(difluoromethyl)-5-fluoro-N-(2-isopropylbenzyl)-1-methyl-1H-pyrazole-4-carboxamide in 500 ml of dioxane are heated at 100 °C for 2 hours. 50 ml of water are then added and the reaction mixture is further heated at 100 °C for another hour. The cooled reaction mixture is filtered over a basic alumina cartridge. The cartridge is washed by dichloromethane and the combined organic extracts are dried over magnesium sulfate and concentrated under vacuum to yield 55.3 g of an orange oil. The residue is triturated with a few mL of diethyl-ether until crystallisation occurs. Crystals are filtered off and dried under vacuum at 40 °C for 15 hours to yield 46.8 g (88% yield) of N-cyclopropyl-3-(difluoromethyl)-5-fluoro-N-(2-isopropylbenzyl)-1-methyl-1H-pyrazole-4-carbothioamide. Mp = 64-70 °C. Table 1 provides the logP and NMR data ($^1$H) of compounds A1 to A20.

In table 1, the logP values were determined in accordance with EEC Directive 79/831 Annex V.A8 by HPLC (High Performance Liquid Chromatography) on a reversed-phase column (C 18), using the method described below : Temperature: 40 °C ; Mobile phases : 0.1% aqueous formic acid and acetonitrile ; linear gradient from 10% acetonitrile to 90% acetonitrile.

Calibration was carried out using unbranched alkan-2-ones (comprising 3 to 16 carbon atoms) with known logP values (determination of the logP values by the retention times using linear interpolation between two successive alkanones). lambda-max-values were determined using UV-spectra from 200 nm to 400 nm and the peak values of the chromatographic signals.

| Cmpd | logP | NMR |
|---|---|---|
| A1 | 3.35 | $^1$H NMR (500 MHz, $CHCl_3$-d): δ ppm 0.64 (bs, 4H), 1.21 (d, J=6.60 Hz, 6H), 2.44 - 2.80 (m, 1H), 3.01 - 3.29 (m, 1H), 3.78 (s, 3H), 4.76 (bs, 2H), 6.89 (t, J=54.70 Hz, 1H), 7.12 - 7.33 (m, 4H). |

| Cmpd | logP | NMR |
|---|---|---|
| A2 | 3.44 | $^1H$ NMR (500 MHz, $CHCl_3$-d): δ ppm 0.47 - 0.77 (m, 6H), 0.80 - 1.04 (m, 2H), 1.92 (bs, 1H), 2.66 (bs, 1H), 3.80 (s, 3H), 4.92 (bs, 2H), 6.90 (t, J=54.50 Hz, 1H), 7.01 - 7.25 (m, 4H). |
| A3 | 4.06 | $^1H$ NMR (500 MHz, $CHCl_3$-d): δ ppm 0.61 (bs, 4H), 1.46 (s, 9H), 2.77 - 2.98 (m, 1H), 3.89 (s, 3H), 5.05 (bs, 2 H), 6.91 (t, J=54.70 Hz, 1H), 7.20 (bs, 3H), 7.35 - 7.48 (m, 1H). |
| A4 | 3.76 | $^1H$ NMR (300 MHz, $CHCl_3$-d): δ ppm 0.65 - 0.69 (m, 4H), 1.21 (t, 3H), 2.62 - 2.64 (m, 3H), 3.81 (s, 3H), 4.70 (s, 2H), 6.85 (t, J=54.6 Hz, 1H), 7.04 - 7.22 (m, 3H). |
| A5 | 4.09 | $^1H$ NMR (500 MHz, $CHCl_3$-d): δ ppm 0.63 - 0.73 (m, 4H), 1.22 (d, J=6.92 Hz, 6H), 2.59 - 2.87 (m, 1H), 2.98 - 3.30 (m, 1H), 3.82 (s, 3H), 4.74 (bs, 2H), 6.88 (t, J=54.40 Hz, 1H), 7.20 - 7.27 (m, 3H). |
| A6 | 3.41 | $^1H$ NMR (300 MHz, $CHCl_3$-d): δ ppm 0.65 - 0.66 (m, 4H), 1.21 (t, 3H), 2.62 (q, 2H), 2.64 (bs, 1H), 3.81 (s, 3H), 4.71 (s, 2H), 6.86 (t, J=54.6 Hz, 1H), 6.89 - 6.95 (m, 2H), 7.13 - 7.18 (m, 1H). |
| A7 | 3.70 | $^1H$ NMR (300 MHz, $CHCl_3$-d): δ ppm 0.65 - 0.69 (m, 4H), 1.22 (d, 6H), 2.69 (bs, 1H), 3.10 - 3.14 (m, 1H), 3.81 (s, 3H), 4.75 (s, 2H), 6.86 (t, J=54.6 Hz, 1H), 6.88 - 6.93 (m, 2H), 7.23 - 7.28 (m, 1H). |
| A8 | 3.46 | $^1H$ NMR (300 MHz, $CHCl_3$-d): δ ppm 0.60 - 0.66 (m, 6H), 0.89 - 0.95 (m, 2H), 1.82 - 1.84 (m, 1H), 2.73 (bs, 1H), 3.81 (s, 3H), 4.89 (s, 2H), 6.68 - 6.99 (m, 4H). |
| A9 | 4.21 | $^1H$ NMR (300 MHz, $CHCl_3$-d): δ ppm 0.64 - 0.68 (m, 4H), 1.56-1.62 (m, 2H), 1.62 - 1.70 (m, 2H), 1.76 - 1.83 (m, 2H), 1.96 - 2.05 (m, 2H), 2.71 (bs, 1H), 3.13 - 3.19 (m, 1H), 3.81 (s, 3H), 4.76 (s, 2H), 6.86 (t, J=54.0 Hz, 1H), 6.87 - 6.97 (m, 2H), 7.23 - 7.28 (m, 1H). |
| A10 | 3.65 | $^1H$ NMR (400 MHz, $CHCl_3$-d): δ ppm 0.65 (bs, 4H), 1.21 (d, J=6.75 Hz, 5H), 2.29 - 2.59 (m, 1H), 3.00 - 3.36 (m, 1H), 3.79 (s, 3H), 4.83 (s, 2H), 6.68 - 7.06 (m, 2H), 7.13 (d, J=7.78 Hz, 1H), 7.27 - 7.33 (m, 1H). |
| A11 | 3.70 | $^1H$ NMR (500 MHz, $CHCl_3$-d): δ ppm 0.65 (bs, 4H), 2.31 (s, 3H), 2.64 (m, 1H), 3.81 (s, 3H), 4.73 (bs, 2H), 6.89 (t, J=54.6 Hz, 1H), 7.01-7.14 (m, 3H). |
| A12 | 3.99 | $^1H$ NMR (500 MHz, $CHCl_3$-d): δ ppm 0.66 (bs, 4H), 1.22 (d, J=6.97 Hz, 6H), 2.31 (s, 3H), 2.54 - 2.75 (m, 1H), 2.99 - 3.25 (m, 1H), 3.81 (s, 3H), 4.75 (bs, 2H), 6.89 (t, J=53.90Hz, 1H), 7.01 - 7.23 (m, 3H). |
| A13 | 3.76 | $^1H$ NMR (500 MHz, $CHCl_3$-d): δ ppm 0.61 - 0.68 (m, 6H), 0.80 - 1.00 (m, 2H), 1.74 - 2.00 (m, 1H), 2.31 (s, 3H), 2.53 - 2.82 (m, 1H), 3.81 (s, 3H), 4.89 (bs, 2H), 6.83 (t, J=54.80 Hz, 1H), 6.91 - 7.06 (m, 3H). |

| Cmpd | logP | NMR |
|---|---|---|
| A14 | 4.36 | $^1H$ NMR (500 MHz, $CHCl_3$-d): δ ppm 0.62 (m, 4H), 1.44 (s, 9H), 2.28 (s, 3H), 2.74 - 3.02 (m, 1H), 3.83 (bs, 3H), 5.02 (bs, 2H), 6.85 (t, J=54.40 Hz, 1 H), 7.01 (bs, 1H), 7.21 - 7.29 (m, 2 H). |
| A15 | 3.80 | $^1H$ NMR (500 MHz, $CHCl_3$-d): δ ppm 0.50 - 0.67 (m, 4H), 2.81 (bs, 1H), 3.78 (s, 3H), 4.85 (bs, 2H), 6.78 (t, J=55.00 Hz, 1H), 7.20 - 7.29 (m, 2H), 7.54 (d, J=8.17 Hz, 1H). |
| A16 | 3.78 | $^1H$ NMR (500 MHz, $CHCl_3$-d): δ ppm 0.55 - 0.70 (m, 4H), 2.37 (s, 3H), 2.72 - 3.04 (m, 1H), 3.83 (bs, 3H), 4.91 (bs, 2H), 6.86 (t, J=54.50 Hz, 1H), 7.10 - 7.20 (m, 2H), 7.54 (d, J=7.89 Hz, 1H). |
| A17 | 3.46 | $^1H$ NMR (500 MHz, $CHCl_3$-d): δ ppm 0.47 - 0.64 (m, 4H), 2.29 - 2.55 (m, 1H), 3.80 (s, 3H), 5.05 (s, 2H), 6.95 (t, J=54.40 Hz, 1H), 7.40 (t, J=7.86 Hz, 1H), 7.60 - 7.70 (dd, 2H). |
| A18 | 3.62 | $^1H$ NMR (500 MHz, $CHCl_3$-d): δ ppm 0.50 - 0.74 (m, 4H), 2.45 - 2.71 (m, 1H), 3.81 (s, 3H), 4.99 (s, 2H), 6.91 (t, J=54.40 Hz, 1H), 7.45 - 7.57 (m, 2H). |
| A19 | 4.04 | $^1H$ NMR (500 MHz, $CHCl_3$-d): δ ppm 0.65 (bs, 4H), 1.20 (t, J=7.43 Hz, 3H), 2.22 (s, 3H), 2.24 (s, 3H), 2.58 - 2.64 (m, 2H), 3.80 (s, 3H), 4.70 (bs, 2H), 6.89 (t, J=54.70 Hz, 3H), 6.98 (bs, 2H). |
| A20 | 4.36 | $^1H$ NMR (500 MHz, $CHCl_3$-d): δ ppm 0.55 - 0.84 (m, 4H), 1.27 (d, J=6.97 Hz, 6H), 2.73 - 2.85 (m, 1H), 3.04 - 3.23 (m, 1H), 3.80 (s, 3H), 4.60 - 5.06 (m, 1H), 6.99 - 7.38 (m, 5H). |

Biological examples:

Seeds of monocotyledonous and dicotyledonous crop plants were placed in sandy loam in wood-fiber pots, covered with soil and cultivated in a greenhouse under good growth conditions. The test plants were treated at the early leaf stage (BBCH10 - BBCH13). To ensure uniform water supply before commencement of stress, the potted plants were supplied with the maximum amount of water immediately beforehand by dam irrigation.

The inventive compounds, formulated in the form of wettable powders (WP) were sprayed onto the green parts of the plants as an aqueous suspension at an equivalent water application rate of 600 1/ha with addition of 0.2% wetting agent (agrotin). Substance application was followed immediately by stress treatment of the plants, for which the pots were transferred in plastic inserts in order to prevent subsequent, excessively rapid drying.

Drought stress was induced by gradual drying out under the following conditions:

"day": 14 hours with illumination at ~26°C
"night": 10 hours without illumination at ~18°C

The duration of the respective stress phases was guided mainly by the state of the untreated, stressed control plants and thus varies from crop to crop. It was ended (by re-irrigating or transfer to a greenhouse with good growth conditions) as soon as irreversible damage is observed on the untreated, stressed control plants. In the case of dicotyledonous crops, for example oilseed rape and soya, the duration of the drought stress phase was between 3 and 6 days, in the case of monocotyledonous crops, for example wheat, barley or corn, between 6 and 11 days.

The end of the stress phase was followed by an approx. 5-7-day recovery phase, during which the plants were once again kept under good growth conditions in a greenhouse. In order to rule out any influence of the effects observed by any fungicidal action of the test compounds, it was additionally ensured that the tests proceed without fungal infection and without infection pressure.

After the recovery phase had ended, the intensities of damage were rated visually compared to untreated, unstressed controls of the same age (in the case of drought stress) or the same growth stage (in the case of cold stress). The intensity of damage was first assessed as a percentage (100% = plants have died, 0% = like control plants). These values were then used to calculate the efficacy of the test compounds (= percentage reduction in the intensity of damage as a result of substance application) by the following formula:

$$EF = \frac{(DV_{us} - DV_{ts}) \times 100}{DV_{us}}$$

EF: efficacy (%)
$DV_{us}$: damage value of the untreated, stressed control
$DV_{ts}$: damage value of the plants treated with test compound

The tables below list mean values in each case from 2 or more independent experiments.

Tables A.1 to A.3 show, by way of example, the efficacies of compound of formula (I) as herein defined under drought stress and in conjunction with application to different crop plants, i.e. in table A.1 on application to BRSNS (*Brassica napus*), in table A.2 on application to TRZAS (*Triticum aestivum*) and in table A.3 on application to ZEAMX (*Zea mays*):

Table A.1: BRSNS (*Brassica napus*)

| No. | Substance | Dosage | Unit | EF (BRSNS) |
|---|---|---|---|---|
| Compound A5 | N-(5-chloro-2-isopropylbenzyl)-N-cyclopropyl-3-(difluoromethyl)-5-fluoro-1-methyl-1H-pyrazole-4-carboxamide | 25 | g/ha | >5 |
| | | 25 | g/ha | >5 |
| Compound A2 | N-cyclopropyl-N-(2-cyclopropylbenzyl)-3-(difluoromethyl)-5-fluoro-1-methyl-1H-pyrazole-4-carboxamide | 250 | g/ha | >5 |
| | | 25 | g/ha | >5 |
| | | 2.5 | g/ha | >5 |
| Compound A19 | N-cyclopropyl-3-(difluoromethyl)-N-(2-ethyl-4,5-dimethylbenzyl)-5-fluoro-1-methyl-1H-pyrazole-4-carboxamide | 25 | g/ha | > 5 |
| Compound A6 | N-cyclopropyl-3-(difluoromethyl)-N-(2-ethyl-5-fluorobenzyl)-5-fluoro-1-methyl-1H-pyrazole-4-carboxamide | 25 | g/ha | > 5 |
| Compound A8 | N-cyclopropyl-N-(2-cyclopropyl-5-fluorobenzyl)-3-(difluoromethyl)-5-fluoro-1-methyl- 1H-pyrazole-4-carboxamide | 2.5 | g/ha | > 5 |
| Compound A7 | N-cyclopropyl-3-(difluoromethyl)-5-fluoro-N-(5-fluoro-2-isopropylbenzyl)-1-methyl-1H-pyrazole-4-carboxamide | 250 | g/ha | > 5 |
| Compound A12 | N-cyclopropyl-3-(difluoromethyl)-5-fluoro-N-(2-isopropyl-5-methylbenzyl)-1-methyl-1H-pyrazole-4-carboxamide | 250 | g/ha | >5 |
| | | 25 | g/ha | >5 |
| | | 2.5 | g/ha | > 5 |

Table A.2 : TRZAS (*Triticum aestivum*)

| No. | Substance | Dosage | Unit | EF (TRZAS) |
|---|---|---|---|---|
| Compound A16 | N-cyclopropyl-3 -(difluoromethyl)-5 -fluoro-1-methyl-N-[5-methyl-2- | 250 | g/ha | >5 |
| | (trifluoromethyl)benzyl]-1H-pyrazole-4-carboxamide | | | |

(continued)

| No. | Substance | Dosage | Unit | EF (TRZAS) |
|---|---|---|---|---|
| Compound A5 | N-(5-chloro-2-isopropylbenzyl)-N-cyclopropyl-3-(difluoromethyl)-5-fluoro-1-methyl-1H-pyrazole-4-carboxamide | 250 | g/ha | >5 |
| | | 25 | g/ha | >5 |
| Compound A19 | N-cyclopropyl-3-(difluoromethyl)-N-(2-ethyl-4,5-dimethylbenzyl)-5-fluoro-1-methyl-1H-pyrazole-4-carboxamide | 25 | g/ha | > 5 |
| Compound A3 | N-(2-tert-butylbenzyl)-N-cyclopropyl-3-(difluoromethyl)-5-fluoro-1-methyl-1H-pyrazole-4-carboxamide | 250 | g/ha | >5 |
| | | 25 | g/ha | > 5 |
| Compound A20 | N-cyclopropyl-3-(difluoromethyl)-5-fluoro-N-(2-isopropylbenzyl)- 1 -methyl- 1H-pyrazole-4-carbothioamide | 250 | g/ha | >5 |
| Compound A6 | N-cyclopropyl-3-(difluoromethyl)-N -(2-ethyl-5-fluorobenzyl)-5-fluoro-1-methyl-1H-pyrazole-4-carboxamide | 250 | g/ha | >5 |
| | | 25 | g/ha | > 5 |
| Compound A4 | N-(5-chloro-2-ethylbenzyl)-N-cyclopropyl-3-(difluoromethyl)-5-fluoro-1-methyl-1H-pyrazole-4-carboxamide | 25 | g/ha | > 5 |
| Compound A11 | N-cyclopropyl-3-(difluoromethyl)-N-(2-ethyl-5-methylbenzyl)-5-fluoro-1-methyl-1H-pyrazole-4-carboxamide | 25 | g/ha | > 5 |
| Compound A14 | N-(2-tert-butyl-5-methylbenzyl)-N-cyclopropyl-3-(difluoromethyl)-5-fluoro-1-methyl-1H-pyrazole-4-carboxamide | 250 | g/ha | >5 |

Table A.3 : ZEAMX (*Zea mays*)

| No. | Substance | Dosage | Unit | EF (ZEAMX) |
|---|---|---|---|---|
| Compound A8 | N-cyclopropyl-N-(2- | 250 | g/ha | > 5 |
| | cyclopropyl-5-fluorobenzyl)-3-(difluoromethyl)-5-fluoro-1-methyl-1H-pyrazole-4-carboxamide | | | |

## Claims

**1.** A method for increasing the drought tolerance of plants which are without fungal infection and without infection pressure, comprising applying to said plants, to the seeds from which they grow or to the locus in which they grow, a non-phytotoxic, effective for enhancing the resistance of plants to drought, amount of a compound selected from the group consisting of :

N-cyclopropyl-N-(2-cyclopropylbenzyl)-3-(difluoromethyl)-5-fluoro-1-methyl-1H-pyrazole-4-carboxamide (compound A2),
N-(2-tert-butylbenzyl)-N-cyclopropyl-3-(difluoromethyl)-5-fluoro-1-methyl-1H-pyrazole-4-carboxamide (compound A3),
N-(5-chloro-2-ethylbenzyl)-N-cyclopropyl-3-(difluoromethyl)-5-fluoro-1-methyl-1H-pyrazole-4-carboxamide (compound A4),
N-(5-chloro-2-isopropylbenzyl)-N-cyclopropyl-3-(difluoromethyl)-5-fluoro-1-methyl-1H-pyrazole-4-carboxamide (compound A5),
N-cyclopropyl-3-(difluoromethyl)-N-(2-ethyl-5-fluorobenzyl)-5-fluoro-1-methyl-1H-pyrazole-4-carboxamide (compound A6),
N-cyclopropyl-3-(difluoromethyl)-5-fluoro-N-(5-fluoro-2-isopropylbenzyl)-1-methyl-1H-pyrazole-4-carboxamide (compound A7),
N-cyclopropyl-N-(2-cyclopropyl-5-fluorobenzyl)-3-(difluoromethyl)-5-fluoro-1-methyl-1H-pyrazole-4-carboxamide (compound A8),
N-cyclopropyl-3-(difluoromethyl)-N-(2-ethyl-5-methylbenzyl)-5-fluoro-1-methyl-1H-pyrazole-4-carboxamide (compound A11),
N-cyclopropyl-3-(difluoromethyl)-5-fluoro-N-(2-isopropyl-5-methylbenzyl)-1-methyl-1H-pyrazole-4-carboxamide (compound A12),
N-(2-tert-butyl-5-methylbenzyl)-N-cyclopropyl-3-(difluoromethyl)-5-fluoro-1-methyl-1H-pyrazole-4-carboxamide (compound A14),
N-cyclopropyl-3-(difluoromethyl)-5-fluoro-1-methyl-N-[5-methyl-2-(trifluoromethyl)benzyl]-1H-pyrazole-4-carboxamide (compound A16),
N-cyclopropyl-3-(difluoromethyl)-N-(2-ethyl-4,5-dimethylbenzyl)-5-fluoro-1-methyl-1H-pyrazole-4-carboxamide (compound A19), and
N-cyclopropyl-3-(difluoromethyl)-5-fluoro-N-(2-isopropylbenzyl)-1-methyl-1H-pyrazole-4-carbothio-amide (compound A20);
or an agrochemically acceptable salt thereof,
wherein said compound is applied to said plants or the locus in which they grow at an application rate of from 0.001 kg/ha to 2 kg/ha or as seed treatment at an application rate of from 0.001 to 250 g/kg of seeds.

**2.** A method according to claim 1, wherein the compound is N-(5-chloro-2-isopropylbenzyl)-N-cyclopropyl-3-(difluoromethyl)-5-fluoro-1-methyl-1H-pyrazole-4-carboxamide or an agrochemically acceptable salt thereof.

**3.** A method according to claim 1 or 2, wherein the compound is applied to said plants or the locus in which they grow at an application rate of from 0.005 kg/ha to 0.5 kg/ha.

**4.** A method according to any one of claims 1 to 3, wherein the plants being selected from the group consisting of cereals, cotton, vine, maize, soybean, oilseed rape, sunflower, turf, horticultural crops, shrubs, fruit-trees, fruit-plants, vegetables.

**5.** A method according to any one of claim 1 to 4 wherein the plants are selected among cereals, oilseeds rape and corn.

**6.** A method according to any one of claim 1 to 5 wherein the compound is applied in spray application to plants and plant parts in combinations with one or more active ingredients selected from the group consisting of insecticides, attractants, acaricides, fungicides, nematicides, herbicides, growth regulators, safeners, substances which influence plant maturity and bactericides.

**7.** A method according to any one of claim 1 to 6 wherein the compound is applied in spray application to plants and plant parts in combinations with fertilizers.

**8.** A method according to any one of claim 1 to 7 wherein the compound is applied for application to genetically modified cultivars, the seed thereof, or to cultivated areas on which these cultivars grow.

**Patentansprüche**

**1.** Verfahren zur Erhöhung der Dürretoleranz von Pflanzen ohne Pilzinfektion und ohne Infektionsdruck, bei dem man auf die Pflanzen, auf die Samen, aus denen sie heranwachsen, oder auf den Standort, an dem sie wachsen, eine nichtphytotoxische, zur Steigerung der Widerstandsfähigkeit von Pflanzen gegenüber Dürre wirksame Menge einer Verbindung aus der Gruppe bestehend aus:

N-Cyclopropyl-N-(2-cyclopropylbenzyl)-3-(difluormethyl)-5-fluor-1-methyl-1H-pyrazol-4-carboxamid (Verbindung A2),
N-(2-tert.-Butylbenzyl)-N-cyclopropyl-3-(difluormethyl)-5-fluor-1-methyl-1H-pyrazol-4-carboxamid (Verbindung A3),
N-(5-Chlor-2-ethylbenzyl)-N-cyclopropyl-3-(difluormethyl)-5-fluor-1-methyl-1H-pyrazol-4-carboxamid (Verbindung A4),
N-(5-Chlor-2-isopropylbenzyl)-N-cyclopropyl-3-(difluormethyl)-5-fluor-1-methyl-1H-pyrazol-4-carboxamid (Verbindung A5),

N-Cyclopropyl-3-(difluormethyl)-N-(2-ethyl-5-fluorbenzyl)-5-fluor-1-methyl-1H-pyrazol-4-carboxamid (Verbindung A6),
N-Cyclopropyl-3-(difluormethyl)-5-fluor-N-(5-fluor-2-isopropylbenzyl)-1-methyl-1H-pyrazol-4-carboxamid (Verbindung A7),
N-Cyclopropyl-N-(2-cyclopropyl-5-fluorbenzyl)-3-(difluormethyl)-5-fluor-1-methyl-1H-pyrazol-4-carboxamid (Verbindung A8),
N-Cyclopropyl-3-(difluormethyl)-N-(2-ethyl-5-methylbenzyl)-5-fluor-1-methyl-1H-pyrazol-4-carboxamid (Verbindung A11),
N-Cyclopropyl-3-(difluormethyl)-5-fluor-N-(2-isopropyl-5-methylbenzyl)-1-methyl-1H-pyrazol-4-carboxamid (Verbindung A12),
N-(2-tert.-Butyl-5-methylbenzyl)-N-cyclopropyl-3-(difluormethyl)-5-fluor-1-methyl-1H-pyrazol-4-carboxamid (Verbindung A14),
N-Cyclopropyl-3-(difluormethyl)-5-fluor-1-methyl-N-[5-methyl-2-(trifluormethyl)benzyl]-1H-pyrazol-4-carboxamid (Verbindung A16),
N-Cyclopropyl-3-(difluormethyl)-N-(2-ethyl-4,5-dimethylbenzyl)-5-fluor-1-methyl-1H-pyrazol-4-carboxamid (Verbindung A19) und
N-Cyclopropyl-3-(difluormethyl)-5-fluor-N-(2-isopropylbenzyl)-1-methyl-1H-pyrazol-4-carbothioamid (Verbindung A20)
oder eines agrochemisch unbedenklichen Salzes davon aufbringt,
wobei die Verbindung auf die Pflanzen oder den Standort, an dem sie wachsen, in einer Aufwandmenge von 0,001 kg/ha bis 2 kg/ha oder als Saatgutbehandlung in einer Aufwandmenge von 0,001 bis 250 g/kg Saatgut aufgebracht wird.

**2.** Verfahren nach Anspruch 1, wobei es sich bei der Verbindung um N-(5-Chlor-2-isopropylbenzyl)-N-cyclopropyl-3-(difluormethyl)-5-fluor-1-methyl-1H-pyrazol-4-carboxamid oder ein agrochemisch unbedenkliches Salz davon handelt.

**3.** Verfahren nach Anspruch 1 oder 2, wobei die Verbindung auf die Pflanzen oder den Standort, an dem sie wachsen, in einer Aufwandmenge von 0,005 kg/ha bis 0,5 kg/ha ausgebracht wird.

**4.** Verfahren nach einem der Ansprüche 1 bis 3, wobei die Pflanzen aus der Gruppe bestehend aus Getreide, Baumwolle, Rebe, Mais, Sojabohne, Raps, Sonnenblume, Rasen, gärtnerischen Kulturpflanzen, Sträuchern, Obstbäumen, Obstpflanzen und Gemüsen ausgewählt sind.

**5.** Verfahren nach einem der Ansprüche 1 bis 4, wobei die Pflanzen aus der Gruppe bestehend aus Getreide, Raps und Mais ausgewählt sind.

**6.** Verfahren nach einem der Ansprüche 1 bis 5, bei dem die Verbindung in Sprühapplikation auf Pflanzen und Pflanzenteile in Kombinationen mit einem oder mehreren Wirkstoffen aus der Gruppe bestehend aus Insektiziden, Lockstoffen, Akariziden, Fungiziden, Nematiziden, Herbiziden, Wachstumsregulatoren, Safenern, Stoffen, die die Pflanzenreife beeinflussen, und Bakteriziden aufgebracht wird.

**7.** Verfahren nach einem der Ansprüche 1 bis 6, wobei die Verbindung in Sprühapplikation auf Pflanzen und Pflanzenteile in Kombinationen mit Düngemitteln aufgebracht wird.

**8.** Verfahren nach einem der Ansprüche 1 bis 7, wobei die Verbindung für die Anwendung auf genetisch modifizierte Sorten, deren Saatgut oder auf Anbauflächen, auf denen diese Sorten wachsen, aufgebracht wird.

## Revendications

**1.** Procédé pour l’augmentation de la tolérance à la sécheresse de végétaux qui sont sans infection fongique et sans pression infectieuse, comprenant l’application auxdites végétaux, aux graines à partir desquelles ils poussent ou au site dans lequel ils poussent, d’une quantité non phytotoxique, efficace pour l’amélioration de la résistance de végétaux à la sécheresse, d’un composé choisi dans le groupe constitué par :

N-cyclopropyl-N-(2-cyclopropylbenzyl)-3-(difluorométhyl)-5-fluoro-1-méthyl-1H-pyrazole-4-carboxamide (composé A2),

N-(2-tert-butylbenzyl)-N-cyclopropyl-3-(difluorométhyl)-5-fluoro-1-méthyl-1H-pyrazole-4-carboxamide (composé A3),
N-(5-chloro-2-éthylbenzyl)-N-cyclopropyl-3-(difluorométhyl)-5-fluoro-1-méthyl-1H-pyrazole-4-carboxamide (composé A4),
N-(5-chloro-2-isopropylbenzyl)-N-cyclopropyl-3-(difluorométhyl)-5-fluoro-1-méthyl-1H-pyrazole-4-carboxamide (composé A5),
N-cyclopropyl-3-(difluorométhyl)-N-(2-éthyl-5-fluorobenzyl)-5-fluoro-1-méthyl-1H-pyrazole-4-carboxamide (composé A6),
N-cyclopropyl-3-(difluorométhyl)-5-fluoro-N-(5-fluoro-2-isopropylbenzyl)-1-méthyl-1H-pyrazole-4-carboxamide (composé A7), N-cyclopropyl-N-(2-cyclopropyl-5-fluorobenzyl)-3-(difluorométhyl)-5-fluoro-1-méthyl-1H-pyrazole-4-carboxamide (composé A8),
N-cyclopropyl-3-(difluorométhyl)-N-(2-éthyl-5-méthylbenzyl)-5-fluoro-1-méthyl-1H-pyrazole-4-carboxamide (composé A11),
N-cyclopropyl-3-(difluorométhyl)-5-fluoro-N-(2-isopropyl-5-méthylbenzyl)-1-méthyl-1H-pyrazole-4-carboxamide (composé A12),
N-(2-tert-butyl-5-méthylbenzyl)-N-cyclopropyl-3-(difluorométhyl)-5-fluoro-1-méthyl-1H-pyrazole-4-carboxamide (composé A14),
N-cyclopropyl-3-(difluorométhyl)-5-fluoro-1-méthyl-N-[5-méthyl-2-(trifluorométhyl)benzyl]-1H-pyrazole-4-carboxamide (composé A16), N-cyclopropyl-3-(difluorométhyl)-N-(2-éthyl-4,5-diméthylbenzyl)-5-fluoro-1-méthyl-1H-pyrazole-4-carboxamide (composé A19), et
N-cyclopropyl-3-(difluorométhyl)-5-fluoro-N-(2-isopropylbenzyl)-1-méthyl-1H-pyrazole-4-carbothio-amide (composé A20) ;
ou d'un sel acceptable sur le plan agrochimique correspondant,
ledit composé étant appliqué auxdits végétaux ou
au site dans lequel ils poussent à raison d'un taux d'application allant de 0,001 kg/ha à 2 kg/ha ou en tant que traitement pour des graines à raison d'un taux d'application allant de 0,001 à 250 g/kg de graines.

2. Procédé selon la revendication 1, le composé étant le N-(5-chloro-2-isopropylbenzyl)-N-cyclopropyl-3-(difluorométhyl)-5-fluoro-1-méthyl-1H-pyrazole-4-carboxamide ou un sel acceptable sur le plan agrochimique correspondant.

3. Procédé selon la revendication 1 ou 2, le composé étant appliqué auxdits végétaux ou au site dans lequel ils poussent à raison d'un taux d'application allant de 0,005 kg/ha à 0,5 kg/ha.

4. Procédé selon l'une quelconque des revendications 1 à 3, les végétaux étant choisis dans le groupe constitué par des céréales, le coton, la vigne, le maïs, les fèves de soja, le colza, le tournesol, le gazon, des cultures horticoles, des arbustes, des arbres fruitiers, des plantes fruitières, des légumes.

5. Procédé selon l'une quelconque des revendications 1 à 4, les végétaux étant choisis parmi des céréales, le colza et le maïs.

6. Procédé selon l'une quelconque des revendications 1 à 5, le composé étant appliqué par application par pulvérisation à des végétaux et des parties de végétaux en combinaison avec un ou plusieurs ingrédients actifs choisis dans le groupe constitué par des insecticides, des substances attractives, des acaricides, des fongicides, des nématicides, des herbicides, des régulateurs de croissance, des phytoprotecteurs, des substances qui influencent la maturité végétale et des bactéricides.

7. Procédé selon l'une quelconque des revendications 1 à 6, le composé étant appliqué par application par pulvérisation à des végétaux et des parties de végétaux en combinaison avec des engrais.

8. Procédé selon l'une quelconque des revendications 1 à 7, le composé étant appliqué pour une application à des cultivars génétiquement modifiés, aux graines de ceux-ci, ou à des zones cultivées sur lesquelles ces cultivars poussent.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007087906 A
- WO 2009016220 A
- WO 2010130767 A
- EP 2251331 A
- EP 2012001676 W
- EP 2012001674 W
- WO 200028055 A
- US 5201931 A
- DE 3534948, Draber
- DE 4103253, Bergmann
- DD 277832, Bergmann
- DD 277835, Bergmann
- WO 0004173 A
- WO 04090140 A
- US 20110196000 A1
- DE 4128828 A
- DE 1905834 A
- DE 19631764 A
- US 765491
- US 11765494 B
- US 10926819 B
- US 10782020 B
- US 12032479 B
- US 10783417 B
- US 10782096 B
- US 11657964 B
- US 12192904 B
- US 11396808 B
- US 12166253 B
- US 12166239 B
- US 12166124 B
- US 12166209 B
- US 11762886 B
- US 12364335 B
- US 11763947 B
- US 12252453 B
- US 12209354 B
- US 12491396 B
- US 12497221 B
- US 12644632 B
- US 12646004 B
- US 12701058 B
- US 12718059 B
- US 12721595 B
- US 12638591 B
- WO 11002992 A
- WO 11014749 A
- WO 11103247 A
- WO 11103248 A
- WO 9205251 A
- WO 9509910 A
- WO 9827806 A
- WO 05002324 A
- WO 06021972 A
- US 6229072 B
- WO 8910396 A
- WO 9102069 A
- WO 0166704 A
- EP 0837944 A
- WO 0066746 A
- WO 0066747 A
- WO 0226995 A
- WO 11000498 A
- US 5776760 A
- US 5463175 A
- WO 0236782 A
- WO 03092360 A
- WO 05012515 A
- WO 07024782 A
- WO 01024615 A
- WO 03013226 A
- US 517991
- US 10739610 B
- US 12139408 B
- US 12352532 B
- US 11312866 B
- US 11315678 B
- US 12421292 B
- US 11400598 B
- US 11651752 B
- US 11681285 B
- US 11605824 B
- US 12468205 B
- US 11760570 B
- US 11762526 B
- US 11769327 B
- US 11769255 B
- US 11943801 B
- US 12362774 B
- US 588811
- US 11185342 B
- US 12364724 B
- US 11185560 B
- US 12423926 B
- US 760602
- US 5561236 A
- US 5648477 A
- US 5646024 A

- US 5273894 A
- US 5637489 A
- US 5276268 A
- US 5739082 A
- US 5908810 A
- US 7112665 B
- WO 9638567 A
- WO 9924585 A
- WO 9924586 A
- WO 2009144079 A
- WO 2002046387 A
- US 6768044 B
- WO 11076877 A
- WO 11076882 A
- WO 11076885 A
- WO 11076889 A
- WO 11076892 A
- WO 9934008 A
- WO 0236787 A
- WO 2004024928 A
- WO 2007103567 A
- WO 2008150473 A
- US 5605011 A
- US 5378824 A
- US 5141870 A
- US 5013659 A
- US 5767361 A
- US 5731180 A
- US 5304732 A
- US 4761373 A
- US 5331107 A
- US 5928937 A
- WO 9633270 A
- WO 2004040012 A
- WO 2004106529 A
- WO 2005020673 A
- WO 2005093093 A
- WO 2006007373 A
- WO 2006015376 A
- WO 2006024351 A
- WO 2006060634 A
- WO 11076345 A
- WO 2012058223 A
- US 61288958
- US 5084082 A
- WO 9741218 A
- US 5773702 A
- WO 99057965 A
- US 5198599 A
- WO 01065922 A
- EP 1999141 A
- WO 2007107302 A
- US 249016
- US 214022
- EP 08010791
- WO 2007027777 A
- WO 9421795 A
- US 61126083
- US 61195019 B
- WO 2007080126 A
- WO 2006129204 A
- WO 2007074405 A
- WO 2007080127 A
- WO 2007035650 A
- WO 2006045633 A
- EP 04077984
- EP 06009836
- WO 2004090140 A
- EP 04077624
- WO 2006133827 A
- EP 07002433 W
- EP 1999263 A
- WO 2007107326 A
- EP 0571427 A
- WO 9504826 A
- EP 0719338 A
- WO 9615248 A
- WO 9619581 A
- WO 9627674 A
- WO 9711188 A
- WO 9726362 A
- WO 9732985 A
- WO 9742328 A
- WO 9744472 A
- WO 9745545 A
- WO 9827212 A
- WO 9840503 A
- WO 9958688 A
- WO 9958690 A
- WO 9958654 A
- WO 0008184 A
- WO 0008185 A
- WO 0008175 A
- WO 0028052 A
- WO 0077229 A
- WO 0112782 A
- WO 0112826 A
- WO 02101059 A
- WO 03071860 A
- WO 2004056999 A
- WO 2005030942 A
- WO 2005030941 A
- WO 2005095632 A
- WO 2005095617 A
- WO 2005095619 A
- WO 2005095618 A
- WO 2005123927 A
- WO 2006018319 A
- WO 2006103107 A
- WO 2006108702 A
- WO 2007009823 A
- WO 0022140 A
- WO 2006063862 A
- WO 2006072603 A
- WO 02034923 A
- EP 06090134

- EP 06090228
- EP 06090227
- EP 07090007
- EP 07090009
- WO 0114569 A
- WO 0279410 A
- WO 0333540 A
- WO 2004078983 A
- WO 0119975 A
- WO 9526407 A
- WO 9634968 A
- WO 9820145 A
- WO 9912950 A
- WO 9966050 A
- WO 9953072 A
- US 6734341 B
- WO 0011192 A
- WO 9822604 A
- WO 9832326 A
- WO 0198509 A
- WO 2005002359 A
- US 5824790 A
- US 6013861 A
- WO 9404693 A
- WO 9409144 A
- WO 9411520 A
- WO 9535026 A
- WO 9720936 A
- WO 10012796 A
- WO 10003701 A
- EP 0663956 A
- WO 9601904 A
- WO 9621023 A
- WO 9839460 A
- WO 9924593 A
- WO 9531553 A
- US 2002031826 A
- US 6284479 B
- US 5712107 A
- WO 9747806 A
- WO 9747807 A
- WO 9747808 A
- WO 0014249 A
- WO 0073422 A
- WO 0047727 A
- EP 06077301
- US 5908975 A
- EP 0728213 A
- WO 2006032538 A
- WO 2007039314 A
- WO 2007039315 A
- WO 2007039316 A
- JP 2006304779 B
- WO 2005012529 A
- US 020360
- US 61054026
- WO 11095528 A
- WO 9800549 A
- WO 2004053219 A
- WO 0117333 A
- WO 0245485 A
- WO 2005017157 A
- EP 08075514
- US 61128938 F
- WO 2006136351 A
- WO 11089021 A
- WO 2012074868 A
- US 5969169 A
- US 5840946 A
- US 6323392 B
- US 6063947 A
- US 6270828 B
- US 6169190 B
- US 5965755 A
- WO 11060946 A
- US 5434283 A
- US 668303
- WO 2012075426 A
- US 61135230
- WO 09068313 A
- WO 10006732 A
- WO 2012090499 A
- WO 10121818 A
- WO 10145846 A
- WO 2006128569 A
- WO 2006128570 A
- US 2002120964 A
- WO 2002034946 A
- WO 2010117737 A
- WO 2010117735 A
- WO 2005103266 A
- US 2005216969 A
- US 2007143876 A
- WO 2006098952 A
- US 2006230473 A
- WO 2011075593 A
- WO 2011075595 A
- WO 2010077816 A
- US 2006162007 A
- WO 2004053062 A
- US 2003126634 A
- WO 2010080829 A
- US 2009217423 A
- WO 2006128573 A
- US 20100024077 A
- WO 2006128571 A
- WO 2006128572 A
- US 2006130175 A
- WO 2004039986 A
- US 2007067868 A
- WO 2005054479 A
- WO 2005054480 A
- WO 2011022469 A
- US 2006070139 A
- WO 2009100188 A
- WO 2011066384 A

- WO 2011066360 A
- US 2009137395 A
- WO 2008112019 A
- US 2008312082 A
- WO 2008054747 A
- US 20090210970 A
- WO 2009103049 A
- US 20100184079 A
- WO 2008002872 A
- WO 2007091277 A
- US 2006059581 A
- WO 1998044140 A
- US 2005086719 A
- US 2005188434 A
- WO 2008151780 A
- US 2010050282 A
- WO 2007017186 A
- WO 2010076212 A
- US 2004172669 A
- WO 2004074492 A
- US 2008064032 A
- WO 2006108674 A
- US 2008320616 A
- WO 2006108675 A
- US 2008196127 A
- WO 2003013224 A
- US 2003097687 A
- US 6468747 B
- WO 2000026345 A
- US 20082289060 A
- WO 2000026356 A
- US 2007028322 A
- WO 2005061720 A
- US 2009300784 A
- WO 2007142840 A
- US 2008167456 A
- WO 2005103301 A
- US 2004250317 A
- WO 2002100163 A
- US 2002102582 A
- WO 2004011601 A
- US 2006095986 A
- WO 2011062904 A
- WO 2009111263 A
- US 20110138504 A
- US 2009130071 A
- WO 2009064652 A
- US 20100080887 A
- WO 2010037016 A
- WO 2011034704 A
- WO 2010024976 A
- US 20110067141 A
- WO 2009102873 A
- US 2008028482 A
- WO 2005059103 A
- WO 2004072235 A
- US 2006059590 A
- WO 2007140256 A
- US 2008260932 A
- US 2006282915 A
- WO 2006130436 A
- WO 2001031042 A
- WO 2001041558 A
- US 2003188347 A
- US 2007292854 A
- WO 2008114282 A
- WO 2002036831 A
- US 2008070260 A
- WO 200244407 A
- US 2009265817 A
- US 2001029014 A
- WO 2001051654 A
- US 2010077501 A
- WO 2008122406 A
- WO 2006128568 A
- US 2005039226 A
- WO 2004099447 A
- WO 2003052073 A
- WO 2011153186 A1
- WO 2011084621 A
- WO 2011063413 A2
- WO 2011066360 A1
- WO 2011066384 A1
- WO 2011075593 A1
- WO 2011075595 A1
- WO 2011084621 A1
- WO 2011084632 A1
- WO 2012033794 A2
- WO 2012051199 A2
- WO 2012075426 A1
- WO 2012075429 A1
- WO 2012082548 A2
- WO 2012071039 A1
- US 2012131692 A
- WO 2012075426 A2
- WO 2012075429 A2
- WO 2009140769 A

**Non-patent literature cited in the description**


- Pflanzenbiochemie. Plant Biochemistry. Spektrum Akademischer Verlag, 393-462
- **HANS W. HELDT.** Biochemistry and Molecular Biology of Plants. American Society of Plant Physiologists, 1996, 1102-1203
- **JAGLO-OTTOSEN et al.** *Science,* 1998, vol. 280, 104-106
- **HASEGAWA et al.** *Annu Rev Plant Physiol Plant Mol Biol,* 2000, vol. 51, 463-499

- **INGRAM ; BARTELS.** *Annu Rev Plant Physiol Plant Mol Biol,* 1996, vol. 47, 277-403
- **CLOSE.** *Physiol Plant,* 1997, vol. 100, 291-296
- **BRAY.** *Plant Physiol,* 1993, vol. 103, 1035-1040
- **KIRCH et al.** *Plant Mol Biol,* 2005, vol. 57, 315-332
- **YU et al.** *Mol Cells,* 2005, vol. 19, 328-333
- Biochemistry and Molecular Biology of Plants. American Society of Plant Physiologists, 2000, 850-929
- **SEMBDNER ; PARTHIER.** Ann. Rev. Plant Physiol. Plant Mol. Biol. 1993, vol. 44, 569-589
- **CHURCHILL et al.** *Plant Growth Regul,* 1998, vol. 25, 35-45
- **BARTLETT et al.** *Pest Manag Sci,* 2002, vol. 60, 309
- **CEDERGREEN.** *Env. Pollution,* 2008, vol. 156, 1099
- **MORRISON ; ANDREWS.** *J Plant Growth Regul,* 1992, vol. 11, 113-117
- **CHEN et al.** *Plant Cell Environ,* 2000, vol. 23, 609-618
- **DE BLOCK et al.** *The Plant Journal,* 2004, vol. 41, 95
- **LEVINE et al.** *FEBS Lett,* 1998, vol. 440, 1
- Ullmann's Encyclopedia of Industrial Chemistry. Verlagsgesellschaft, 1987, vol. A-10, 323-431
- Ullmann's Encyclopedia of Industrial Chemistry. 1987, vol. A-10, 363-401
- **COMAI et al.** *Science,* vol. 221, 370-371
- **BARRY et al.** *Curr. Topics Plant Physiol.,* 1992, vol. 7, 139-145
- **SHAH et al.** *Science,* 1986, vol. 233, 478-481
- **GASSER et al.** *J. Biol. Chem.,* 1988, vol. 263, 4280-4289
- **TRANEL ; WRIGHT.** *Weed Science,* 2002, vol. 50, 700-712
- **CRICKMORE et al.** *Microbiology and Molecular Biology Reviews,* 1998, vol. 62, 807-813
- **CRICKMORE et al.** *Bacillus,* 2005, http://www.lifesci.sussex.ac.uk/Home/Neil_Crickmore/Bt
- **MOELLENBECK et al.** *Nat. Biotechnol.,* 2001, vol. 19, 668-72
- **SCHNEPF et al.** *Applied Environm. Microbiol.,* 2006, vol. 71, 1765-1774
- Chemie der Pflanzenschutz- and Schädlingsbekämpfungsmittel. **R. WEGLER.** Chemistry of the Crop Protection Compositions and Pesticides. Springer Verlag, 1970, vol. 2, 401-412
- *J. Med. Chem.,* 2012, vol. 55 (1), 169-196
- *Bioorg. Med. Chem.,* 2006, vol. 14, 8506-8518